# EUROPÄISCHE PATENTSCHRIFT

(11) **EP 4 035 605 B1**
(45) Veröffentlichungstag und Bekanntmachung des Hinweises auf die Patenterteilung: **16.04.2025**
(21) Anmeldenummer: 21154092.7
(22) Anmeldetag: 28.01.2021
(51) Int. Cl.: A61B 17/00, A46B 13/00, A46B 13/04, A46B 11/06, A46B 9/02, A61B 17/16, A61B 17/32

(54) **CHIRURGISCHE REINIGUNGSVORRICHTUNG**
SURGICAL CLEANING APPARATUS
DISPOSITIF DE NETTOYAGE CHIRURGICAL

(43) Veröffentlichungstag der Anmeldung: 03.08.2022
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Schultheiss & Sterzel Patentanwälte PartG mbB

(56) Entgegenhaltungen:
- EP-A1- 3 574 852
- EP-A1- 3 741 316
- US-A1- 2003 083 681
- US-A1- 2011 085 845

## Beschreibung

Die Erfindung betrifft eine chirurgische Reinigungsvorrichtung und ein nichtchirurgisches und nicht- therapeutisches Verfahren zum Betreiben einer solchen chirurgischen Reinigungsvorrichtung.

Gegenstand der Erfindung ist dabei insbesondere eine einmalig zu verwendende chirurgische Reinigungsvorrichtung, die sowohl zur Reinigung von infizierten Gelenkendoprothesen als auch von infizierten Knochen- und Weichgewebearealen bestimmt und geeignet ist. Mit der chirurgischen Reinigungsvorrichtung können aber auch andere gebrauchte Werkzeuge und Bestecke, die im Rahmen einer medizinischen Operation (OP) verwendet wurden, gereinigt werden.

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte beziehungsweise nekrotische Gewebe abgetragen. Dieses Abtragen von infiziertem/nekrotischem Gewebe wird als Debridement bezeichnet. Ebenso müssen die explantierten Gelenkendoprothesen gereinigt werden.

In der Medizin sind gegenwärtig mit Druckluft und mit elektrischer Energie betriebene Antriebseinheiten bekannt. Derartige externe Antriebseinheiten sind in Operationssälen (OP-Sälen) vorhanden und können für eine Vielzahl von Zwecken eingesetzt und genutzt werden. In der septischen Knochenchirurgie ist die Reinigung von debridierten Knochen- und Weichgewebearealen bei der Revision von infizierten Gelenkendoprothesen und die Reinigung von infizierten Osteosyntheseplatten durch Lavagierung Stand der Technik. Dafür werden medizinische Spülvorrichtungen, sogenannte Lavage-Systeme, eingesetzt. Als Spülflüssigkeiten sind physiologische Kochsalzlösung, Ringer-Lösung und Ringer-Lactat-Lösung üblich. Das Ziel der Lavagierung besteht in der Entfernung von Resten des infizierten Gewebes, von Biofilmresten, von Blut und von Wundsekret. Lavage-Systeme sind seit langem bekannt. Exemplarisch sind dafür Vorrichtungen gemäß den Druckschriften DE 10 2011 018 708 A1, EP 2 939 699 B1, EP 3 187 208 B1, US 4 583 531 A, US 5 542 918 A, US 5 779 702 A, US 6 059 754 A, US 2005/0084395 A1 und US 2019/0151531 A1.

Aus der EP 3 574 852 A1 ist ein Vakuummotor bekannt, bei dem ein Stößel axial periodisch bewegt wird. Die US 2003/083681 A1 offenbart ein chirurgisches Handstück mit einem rotierenden Schaft, der aus Handstück hervorragt. Aus der US 2011/085845 A1 ist eine mit Wasserkraft angetriebene Bürste zum Spülen von Geschirr bekannt.

Das Debridieren und Reinigen kann durch Wundspülung mit sogenannten Lavage-Systemen sowie durch Ausschneiden, Raspeln, Sägen und auch Bürsten erfolgen. Eine Vorrichtung zum Bürsten und Sägen ist beispielsweise aus der EP 3 741 316 A1 bekannt. Die verwendeten Vorrichtungen sind nach dem Debridement mit Geweberesten und mit mikrobiellen Keimen kontaminiert. Für eine neue Verwendung müssen diese Instrumente sorgfältig gereinigt und anschließend sterilisiert werden. Dabei muss sich das medizinische Personal vor einer Kontamination beziehungsweise einer Infektion durch Übertragung von mikrobiellen Keimen während der Reinigungsarbeiten schützen. Daher ist es wünschenswert, eine kostengünstige chirurgische Reinigungsvorrichtung bereitzustellen, die nach einmaliger Verwendung ohne aufwendige und nicht gefahrlose Reinigungsschritte mit dem üblichen OP-Abfall zusammen entsorgt werden könnte. Es wäre dann besonders aus Gründen des Ressourcen- und Umweltschutzes aber auch aus Kostengründen sinnvoll, wenn für den Antrieb keine Batterien, Akkumulatoren und Elektromotoren notwendig wären, die Buntmetalle enthalten und daher gegebenenfalls aufwendig recycelt werden müssten. Nach einer mechanischen Reinigung oder Bearbeitung muss häufig zusätzlich ein Debridement erfolgen, um entstandene und möglicherweise infektiöse Rückstände wie Späne, die bei der mechanischen Bearbeitung entstanden sind, zu entfernen. Hierfür wird ein weiterer Arbeitsschritt benötigt und alle verwendeten Vorrichtungen müssen anschließend aufwendig desinfiziert beziehungsweise entsorgt werden.

Die Aufgabe der vorliegenden Erfindung besteht also darin, die Nachteile des Stands der Technik zu überwinden. Insbesondere besteht die Aufgabe der Erfindung in der Entwicklung einer einmalig zu verwendenden chirurgischen Reinigungsvorrichtung, die zur Reinigung von infizierten Gelenkendoprothesen und von infizierten Knochen- und Weichgewebearealen bestimmt ist. Diese Vorrichtung soll möglichst einfach und kostengünstig zu fertigen sein. Weiterhin soll die Vorrichtung durch Ethylenoxid oder Gammasterilisation zu sterilisieren sein. Die Reinigungsvorrichtung soll einfach in der Anwendung sein und möglichst wenig notwendige Arbeitsschritte erfordern. Es wäre vorteilhaft, wenn die Reinigungsvorrichtung nach der Verwendung hygienisch durch Verbrennung entsorgt werden kann. Die chirurgische Reinigungsvorrichtung muss, um chirurgisch anwendbar zu sein, steril sein. Die chirurgische Reinigungsvorrichtung soll möglichst nach beendeter Reinigung zusammen mit anderen Klinikabfällen durch Verbrennung entsorgt werden können. Die chirurgische Reinigungsvorrichtung soll durch eine für medizinische Operationen übliche Antriebseinheit angetrieben werden können.

Die der vorliegenden Erfindung zugrundeliegenden Aufgaben werden gelöst durch eine chirurgische Reinigungsvorrichtung gemäß Anspruch 1 und durch ein nicht- chirurgisches und nicht-therapeutisches Verfahren gemäß Anspruch 12. Eine erfindungsgemäße chirurgische Reinigungsvorrichtung weist ein Gehäuse, eine Antriebswelle, wobei die Antriebswelle drehbar in dem Gehäuse gelagert ist, wobei die Antriebswelle ein hinteres Ende zur Verbindung mit einer Antriebseinheit aufweist und wobei die Antriebswelle ein vorderes Ende aufweist, das dem hinteren Ende gegenüberliegend angeordnet ist, eine Vielzahl elastisch verformbarer Borsten, wobei die elastisch verformbaren Borsten an dem vorderen Ende der Antriebswelle angeordnet sind und mit der Antriebswelle verbunden sind, wobei die elastisch verformbaren Borsten zumindest teilweise aus dem Gehäuse hervorstehen und wobei die elastisch verformbaren Borsten sich größtenteils oder alle von der Drehachse der Antriebswelle weg erstrecken, eine Pumpe zum Pumpen einer Flüssigkeit, wobei die Pumpe mit der Antriebswelle verbunden ist und von der Antriebswelle anzutreiben ist, eine Flüssigkeitszuleitung, wobei die Flüssigkeitszuleitung derart mit der Pumpe verbunden ist, dass eine Flüssigkeit durch die Flüssigkeitszuleitung von der Pumpe in die Pumpe ansaugbar ist, eine Flüssigkeitsableitung, wobei die Flüssigkeitsableitung derart mit der Pumpe verbunden ist, dass eine Flüssigkeit von der Pumpe aus der Pumpe heraus in die Flüssigkeitszuleitung drückbar ist, wobei die Flüssigkeitsableitung im Bereich der elastisch verformbaren Borsten zumindest eine Flüssigkeitsaustrittsöffnung aufweist, wobei die Flüssigkeitsableitung durch die zumindest eine Flüssigkeitsaustrittsöffnung in Richtung der elastisch verformbaren Borsten mündet, auf.

Das vordere Ende der Antriebswelle erstreckt sich über eine Länge der Antriebswelle, ist also vorliegend nicht als ein Punkt oder eine Stelle ohne räumliche Ausdehnung zu verstehen.

Dass die elastisch verformbaren Borsten sich zumindest teilweise von der Drehachse der Antriebswelle weg erstrecken, bedeutet, dass die Richtung, in der sich die elastisch verformbaren Borsten von der Drehachse der Antriebswelle weg erstrecken, einen auf die Drehachse der Antriebswelle bezogenen radialen Anteil aufweisen. Die elastisch verformbaren Borsten müssen sich dabei nicht senkrecht von der Drehachse der Antriebswelle weg erstrecken. Es kann aber vorgesehen sein, dass sich die elastisch verformbaren Borsten senkrecht von der Drehachse der Antriebswelle weg erstrecken

Bevorzugt kann vorgesehen sein, dass das vordere Ende der Antriebswelle zumindest teilweise aus dem Gehäuse herausragt. Der aus dem Gehäuse herausragende Teil der Antriebswelle umfasst bevorzugt das vordere Ende der Antriebswelle.

Bevorzugt kann ferner vorgesehen sein, dass die Flüssigkeitsableitung im Bereich der elastisch verformbaren Borsten in die zumindest eine Flüssigkeitsaustrittsöffnung mündet.

Auch kann vorgesehen sein, dass die Flüssigkeitsableitung an der zumindest einen Flüssigkeitsaustrittsöffnung endet.

Es ist erfindungsgemäß bevorzugt, wenn sich die elastisch verformbaren Borsten oder zumindest dreiviertel der elastisch verformbaren Borsten senkrecht (in einem rechten Winkel) von der Drehachse der Antriebswelle weg erstrecken und/oder mit einem Winkel von mindestens 45° von der Drehachse der Antriebswelle weg erstrecken.

Die Antriebswelle ist bevorzugt vollständig oder zumindest am vorderen Ende der Antriebswelle als eine gerade Stange ausgeführt, wobei die Drehachse der Antriebswelle innerhalb der geraden Stange und parallel zur Achse der geraden Stange verläuft.

Die erfindungsgemäße chirurgische Reinigungsvorrichtung zeichnet sich dadurch aus, dass sie keinen Motor und keinen Energiespeicher aufweist. Dadurch kann die chirurgische Reinigungsvorrichtung kostengünstig als hygienisches Wegwerfprodukt für medizinische Anwendungen gefertigt werden und insbesondere aus leicht verbrennbarem Kunststoff bestehen.

Um chirurgisch anwendbar zu sein, ist es wesentlich, dass die chirurgische Reinigungsvorrichtung steril ist oder zumindest alle Oberflächen der Reinigungsvorrichtung sterilisierbar ist, wie beispielsweise durch Gamma-Strahlen-Sterilisation oder durch Sterilisation mit Ethylenoxid. Hierzu kann bevorzugt vorgesehen sein, dass die chirurgischen Reinigungsvorrichtung in einer für Ethylenoxid durchlässigen Verpackung enthalten ist.

Das hintere Ende der Antriebswelle kann als Anschluss zum Verbinden mit einer externen Antriebseinheit ausgebildet sein, insbesondere als eine Vierkantstange, Sechskantstange oder allgemein als Kantstange ausgebildet sein.

Es kann vorgesehen sein, dass das Gehäuse ein Wellengehäuse aufweist und ein Griffstück zum Halten der chirurgischen Reinigungsvorrichtung mit einer Hand aufweist, wobei das Wellengehäuse die Antriebswelle zumindest in einem Bereich zwischen dem vorderen Ende der Antriebswelle und dem Griffstück umschließt und wobei das Wellengehäuse mit dem Griffstück verbunden ist und das Griffstück eine Öffnung für das hinteren Ende der Antriebswelle aufweist.

Hierdurch ist die chirurgische Reinigungsvorrichtung gefahrlos und bequem manuell einsetzbar. Das Wellengehäuse kann neben dem Schutz der darin befindlichen Teile, wie der Antriebswelle und der Flüssigkeitsableitung zusätzlich dazu genutzt werden, einen geschützten Hohlraum für andere Teile der chirurgischen Reinigungsvorrichtung zu bilden.

Des Weiteren kann vorgesehen sein, dass die Antriebswelle durch eine separate Antriebseinheit drehbar und anzutreiben ist, wobei die separate Antriebseinheit hierzu mit dem hinteren Ende der Antriebswelle verbunden oder verbindbar ist, insbesondere lösbar verbunden oder verbindbar ist.

Die separate Antriebseinheit kann eine externe Antriebseinheit sein.

Hierdurch benötigt die chirurgische Reinigungsvorrichtung keinen eigenen Motor und keinen Energiespeicher. Dadurch kann die chirurgische Reinigungsvorrichtung zum einmaligen Gebrauch gefertigt werden und als hygienisches Wegwerfprodukt durch Verbrennen entsorgt werden.

Ferner kann vorgesehen sein, dass die elastisch verformbaren Borsten sich axial und radial von der Antriebswelle weg erstrecken.

Hierdurch kann mit den elastisch verformbaren Borsten sowohl eine radiale mechanische Reinigung als auch eine frontale mechanische Reinigung an der Spitze des vorderen Endes der Antriebswelle und damit an dem vorderen Ende der chirurgischen Reinigungsvorrichtung erfolgen. Dadurch ist es möglich, sowohl plane als auch röhrenförmige Areale, wie zum Beispiel proximale Femura oder röhrenförmige Kavitäten in Implantaten nach septischen Revisionen, zu reinigen.

Es kann auch vorgesehen sein, dass die elastisch verformbaren Borsten den Außendurchmesser des Gehäuses im Bereich der elastisch verformbaren Borsten um mindestens 1 mm, bevorzugt 2 mm bis 4 mm und besonders bevorzugt um 5 mm bis 10 mm überragen.

Hiermit wird erreicht, dass mit der chirurgischen Reinigungsvorrichtung auch Hinterschneidungen noch erreicht und mechanisch gereinigt werden können und die elastisch verformbaren Borsten auch tiefere Bereiche der zu reinigenden Areale erfassen können. Das Griffstück des Gehäuses kann auch einen größeren Außendurchmesser aufweisen, den die elastisch verformbaren Borsten nicht überragen.

Gemäß einer bevorzugten Weiterbildung kann vorgesehen sein, dass die Pumpe eine selbstansaugende Pumpe ist, die unmittelbar mit der Antriebswelle verbunden ist, wobei die selbstansaugende Pumpe in dem Gehäuse angeordnet ist.

Hierdurch wird ein besonders einfacher Aufbau erreicht. Zudem wird so sichergestellt, dass gleichzeitig mit der Drehung der elastisch verformbaren Borsten ein Ausstoß von Flüssigkeit stattfindet, um so die beiden Reinigungswirkungen zu kombinieren. Eine selbstansaugende Pumpe in der chirurgischen Reinigungsvorrichtung hat den Vorteil, dass die chirurgische Reinigungsvorrichtung Spülflüssigkeiten, wie physiologische Kochsalzlösung, Ringer-Lösung oder Ringer-Lactat-Lösung, selbst ansaugen kann und diese dann in Form von Spülflüssigkeitsstrahlen aus der chirurgischen Reinigungsvorrichtung ausgestoßen wird.

Bevorzugt kann auch vorgesehen sein, dass die Pumpe derart mit der Antriebswelle verbunden ist, dass zumindest bei einem Stillstand der Antriebswelle die Flüssigkeitszuleitung in der Pumpe von der Flüssigkeitsableitung flüssigkeitsundurchlässig getrennt ist oder abgeriegelt ist, wobei bevorzugt bei Drehbewegung der Antriebswelle die Flüssigkeitszuleitung flüssigkeitsleitend mit der Flüssigkeitsableitung verbunden ist.

Die Trennung oder Abriegelung kann durch mechanische Blockierung innerhalb der Pumpe erfolgen. Hiermit kann verhindert werden, dass kontaminierte Spülflüssigkeit durch die Flüssigkeitsableitung und die Pumpe in die Flüssigkeitszuleitung vordringen kann und dort ein Reservoir für die Spülflüssigkeit kontaminiert, ohne dass hierfür ein separates oder zusätzliches Ventilsystem notwendig wäre. Dadurch kann die chirurgische Reinigungsvorrichtung als kostengünstiges und hygienisches Wegwerfprodukt aufgebaut werden. Die Pumpe ist mit der Antriebswelle verbunden, die bei Nichtdrehung der Antriebswelle den Flüssigkeitsstrom der Spülflüssigkeit ausgehend von der Flüssigkeitszuleitung durch mechanische Blockierung der Verbindung zwischen der Flüssigkeitszuleitung und der Flüssigkeitsableitung abriegelt und bei Drehbewegung der Welle den Flüssigkeitsstrom von der Flüssigkeitszuleitung zur Flüssigkeitsableitung freigibt. Dadurch ist es möglich, durch den Verzicht von Ventilen und Rückschlagventilen die Vorrichtung einfach und kostengünstig zu halten.

Gemäß einer besonders bevorzugt Ausgestaltung der vorliegenden Erfindung kann vorgesehen sein, dass die Pumpe eine Impellerpumpe ist, die auf der Antriebswelle angeordnet ist, wobei die Impellerpumpe einen Rotor mit Impellerflügeln aufweist, wobei der Rotor mit den Impellerflügeln kraft- und/oder formschlüssig mit der Antriebswelle verbunden ist.

Hierdurch wird ein besonders einfacher und kostengünstiger Aufbau erreicht. Gleichzeitig sind die Flüssigkeitszuleitung und die Flüssigkeitsableitung durch die Impellerflügel in der Impellerpumpe voneinander getrennt und so wird eine Kontamination der Spülflüssigkeit durch verunreinigte gebrauchte Spülflüssigkeit auf einfache Weise und ohne Ventilsystem verhindert.

Des Weiteren kann vorgesehen sein, dass die Flüssigkeitsableitung zumindest bereichsweise durch einen Teil des Gehäuses gebildet ist, der einen Zwischenraum zwischen der Antriebswelle und dem Gehäuse begrenzt, wobei die zumindest eine Flüssigkeitsaustrittsöffnung durch zumindest eine Bohrung in dem Gehäuse oder in einer frontalen Dichtung des Gehäuses im Bereich der elastisch verformbaren Borsten realisiert ist.

Hierdurch kann der Aufbau besonders einfach und kostengünstig gestaltet werden.

Es kann also auch vorgesehen sein, dass der Zwischenraum zwischen dem die Antriebswelle umgebenden Teil des Gehäuses und der Antriebswelle als die Flüssigkeitsableitung ausgebildet ist, wobei eine Bohrung oder mehrere Bohrungen in dem die Antriebswelle umgebenden Teil des Gehäuses als die zumindest eine Flüssigkeitsaustrittsöffnung angeordnet sind.

Zum Erzeugen eines gepulsten Flüssigkeitsstrahls der Spülflüssigkeit kann vorgesehen sein, dass zumindest eine Blende auf der Antriebswelle derart angebracht ist, so dass die zumindest eine Blende die zumindest eine Flüssigkeitsaustrittsöffnung oder wenigstens eine von mehreren Flüssigkeitsaustrittöffnungen bei einer vollständigen Drehung der Antriebswelle um ihre Drehachse wenigstens einmal abdeckt und wenigstens einmal freigibt.

Hierdurch wird erreicht, dass ein Flüssigkeitsstrom, der durch die zumindest eine Flüssigkeitsaustrittsöffnung austritt, mindestens einmal pro Umdrehung der Antriebswelle unterbrochen wird und pulsierende Flüssigkeitsstrahlen entstehen. Hierdurch wird die Reinigungswirkung der chirurgischen Reinigungsvorrichtung verbessert.

Bevorzugt ist die zumindest eine Blende auf der Antriebswelle formschlüssig und/oder kraftschlüssig angebracht.

Zum Entfernen der gebrauchten Spülflüssigkeit kann vorgesehen sein, dass die chirurgische Reinigungsvorrichtung eine Absaugeinrichtung mit zumindest einer Absaugöffnung im Bereich der elastisch verformbaren Borsten aufweist, wobei die Absaugeinrichtung eine Absaugleitung aufweist, die an eine Unterdruckquelle anschließbar ist oder angeschlossen ist, wobei bevorzugt die Absaugleitung durch ein Rohr realisiert ist, wobei das Rohr das Gehäuse zumindest abschnittsweise umschließt.

Hierdurch kann die gebrauchte Spülflüssigkeit abgesaugt und von dem zu reinigenden Objekt entfernt werden.

Dabei kann vorgesehen sein, dass die Absaugeinrichtung an einen Abscheider zum Abscheiden von festen Bestandteilen aus der gebrauchten Spülflüssigkeit angeschlossen oder anschließbar ist und/oder die Unterdruckquelle eine Vakuumpumpe aufweist, die ein Teil der chirurgischen Reinigungsvorrichtung ist und die von der Antriebswelle antreibbar ist.

Hierdurch muss die gebrauchte Spülflüssigkeit nicht auf andere Weise mit einer separaten Vorrichtung entfernt werden.

Es kann des Weiteren vorgesehen sein, dass die chirurgische Reinigungsvorrichtung einen Spritzschutz aufweist, wobei der Spritzschutz die elastisch verformbaren Borsten teilweise abdeckt, wobei bevorzugt der Spritzschutz an dem Gehäuse befestigt ist und entweder gegen das Gehäuse drehbar gelagert ist oder starr mit dem Gehäuse verbunden ist.

Hierdurch kann die Menge an gebrauchter Spülflüssigkeit, die als Spritzwasser in die Umgebung gespritzt wird, reduziert werden. Hierdurch ist ein hygienischeres Arbeiten mit der chirurgischen Reinigungsvorrichtung möglich. Die elastisch verformbaren Borsten können bei einer Drehung der Antriebswelle über eine Innenseite des Spritzschutzes streifen. Hierdurch kann die Spülflüssigkeit und gegebenenfalls anhaftende Rückstände an dem Spritzschutz angestreift werden.

Bevorzugt kann auch vorgesehen sein, dass die chirurgische Reinigungsvorrichtung aus Kunststoff gefertigt ist, wobei bevorzugt die gesamte chirurgische Reinigungsvorrichtung durch Verbrennung rückstandsfrei entsorgbar ist.

Hierdurch kann die chirurgische Reinigungsvorrichtung als hygienisches Wegwerfprodukt im medizinischen Bereich verwendet werden und zudem kostengünstig gefertigt werden.

Es kann vorgesehen sein, dass die zumindest eine Flüssigkeitsaustrittsöffnung derart geformt und derart relativ zu den elastisch verformbaren Borsten angeordnet ist, dass ein durch die zumindest eine Flüssigkeitsaustrittsöffnung ausgestoßener Strahl der Spülflüssigkeit in den Wirkbereich der drehenden elastisch verformbaren Borsten trifft.

Der Wirkbereich der sich drehenden elastisch verformbaren Borsten ist der Bereich, den die freien Enden der elastisch verformbaren Borsten, die einer Verbindung der elastisch verformbaren Borsten zur Antriebswelle gegenüberliegen, bei einer Drehung der elastisch verformbaren Borsten überstreichen. Das ist der Bereich der chirurgischen Reinigungsvorrichtung, der zur mechanischen Reinigung mit Hilfe der elastisch verformbaren Borsten vorgesehen ist.

Durch das Auftreffen des Strahls der Spülflüssigkeit im Wirkbereich der sich drehenden elastisch verformbaren Borsten wird eine besonders gute Reinigungswirkung erzielt, da die von den elastisch verformbaren Borsten mechanisch abgelösten Partikel von dem Strom der Spülflüssigkeit abtransportiert werden können.

Ferner kann vorgesehen sein, dass die chirurgische Reinigungsvorrichtung eine flexible Antriebswellenverlängerung aufweist, wobei die flexible Antriebswellenverlängerung an das hintere Ende der Antriebswelle angeschlossen oder anschließbar ist und wobei die flexible Antriebswellenverlängerung an eine externe Antriebseinheit anschließbar ist, so dass die Antriebswelle mit der externen Antriebseinheit über die flexible Antriebswellenverlängerung gegen das Gehäuse drehbar ist.

Hierdurch kann die externe Antriebseinheit von den elastisch verformbaren Borsten der chirurgischen Reinigungsvorrichtung und damit von dem Ort der Reinigung entfernt platziert werden, so dass die externe Antriebseinheit, die einen Motor enthält, nicht verschmutzt und daher nicht aufwendig gereinigt werden muss.

Die der vorliegenden Erfindung zugrundeliegenden Aufgaben werden auch gelöst durch ein nicht-chirurgisches und nicht-therapeutisches Verfahren zum Betreiben einer chirurgischen Reinigungsvorrichtung, wobei das Verfahren die folgenden Schritte aufweist:
A) Drehen einer Antriebswelle der chirurgischen Reinigungsvorrichtung;
B) Drehen einer Vielzahl von elastisch verformbaren Borsten, die an einem vorderen Ende der Antriebswelle angeordnet sind, durch das Drehen der Antriebswelle;
C) Antreiben einer Pumpe mit der sich drehenden Antriebswelle; und
D) Pumpen einer Spülflüssigkeit mit der Pumpe durch zumindest eine Flüssigkeitsaustrittsöffnung, wobei die Spülflüssigkeit gepulst oder ungepulst in den Bereich der sich drehenden elastisch verformbaren Borsten gesprüht wird, insbesondere in den Wirkbereich der sich drehenden elastisch verformbaren Borsten gesprüht wird.

Das Verfahren kann beispielsweise zur Reinigung einer gebrauchten explantierten Prothese verwendet werden, damit diese anschließend wiederverwendet werden kann. Es ist aber auch möglich, mit dem Verfahren andere neue und gebrauchte Implantate oder neue oder gebrauchte medizinische Instrumente vor deren Verwendung zu reinigen.

Dabei kann vorgesehen sein, dass das Verfahren mit einer oben erfindungsgemäßen chirurgischen Reinigungsvorrichtung durchgeführt wird.

Das Verfahren hat dann die zu der chirurgischen Reinigungsvorrichtung geschilderten Vorteile. Des Weiteren kann vorgesehen sein, dass vor Schritt A) die chirurgische Reinigungsvorrichtung an eine Antriebseinheit angeschlossen wird, wobei die Drehung der Antriebswelle in den Schritten A) bis D) mit der Antriebseinheit angetrieben wird, wobei bevorzugt die Antriebseinheit an ein dem vorderen Ende gegenüberliegendes hinteres Ende der Antriebswelle angeschlossen wird.

Hierdurch wird klargestellt, dass eine externe Antriebseinheit zum Antreiben und Drehen der Antriebswelle der chirurgischen Reinigungsvorrichtung verwendet wird.

Ferner kann vorgesehen sein, dass die gebrauchte Spülflüssigkeit während der Schritte A) bis D) zumindest teilweise mit einer Absaugeinrichtung abgesaugt wird, wobei bevorzugt nach der Absaugung mit einem Abscheider feste Partikel der gebrauchten Spülflüssigkeit von den flüssigen Bestandteilen getrennt werden.

Hierdurch kann die Reinigungswirkung des Verfahrens erhöht werden und eine Kontamination der Umgebung vermindert werden.

Erfindungsgemäß wird das Verfahren nicht zu einer medizinischen oder therapeutischen Behandlung eines Patienten eingesetzt.

Bei erfindungsgemäßen Verfahren ist eine Anwendung nicht patentierbarer medizinischer Verfahren ausgeschlossen. Wie bereits ausgeführt, kann das Verfahren zur Reinigung von OP-Werkzeugen und explantierten Implantaten verwendet werden, die nicht mit dem Körper eines Patienten verbunden sind, so dass dann keine unmittelbar auf den Körper einwirkende medizinische Anwendung vorliegt.

Der Erfindung liegt die überraschende Erkenntnis zugrunde, dass eine chirurgische Reinigungsvorrichtung durch den Antrieb einer einzelnen Antriebswelle sowohl zum Betreiben einer Bürste mit elastisch verformbaren Borsten, als auch gleichzeitig zum Erzeugen eines gepulsten oder auch ungepulsten Stroms einer Spülflüssigkeit im Wirkbereich der elastisch verformbaren Borsten betrieben werden kann, wobei die Antriebswelle mit einer im OP üblichen Antriebseinheit antreibbar ist und die chirurgische Reinigungsvorrichtung derart einfach und kostengünstig im Aufbau ausgeführt werden kann, dass die chirurgische Reinigungsvorrichtung als kostengünstiges Wegwerfprodukt gefertigt werden kann, die eine hygienische Entsorgung nach einmaligem Gebrauch ermöglicht. Die chirurgische Reinigungsvorrichtung kann hierbei als einfacher Aufsatz zur Befestigung an einer externen Antriebseinheit aufgebaut werden. Der erfindungsgemäße Aufbau ermöglicht dabei, dass die chirurgische Reinigungsvorrichtung sehr weitgehend oder vollständig aus Kunststoff herstellbar ist, wodurch eine hygienische Entsorgung durch Verbrennung mit andere OP-Abfällen ermöglicht wird.

Gleichzeitig wird der Arbeitsablauf bei der Anwendung der chirurgischen Reinigungsvorrichtung dadurch vereinfacht, dass die Schritte des mechanischen Entfernens von Rückständen und des Spülens der gereinigten Oberfläche mit Spülflüssigkeit zur gleichen Zeit erfolgen kann. Dabei wird die Reinigungswirkung erhöht. Insbesondere bei der Verwendung einer flexiblen Antriebswellenverlängerung kann die externe Antriebseinheit derart weit von dem zu reinigenden Areal angeordnet werden, dass eine Verschmutzung der Antriebseinheit verhindert werden kann. Durch diese Maßnahmen kann ein aufwendiges Reinigen der chirurgischen Reinigungsvorrichtung und der externen Antriebseinheit entfallen und dadurch das Risiko einer nicht ausreichenden Reinigung und dadurch das Risiko einer gefährlichen Infektion bei erneuter Anwendung der chirurgischen Reinigungsvorrichtung und der Antriebseinheit nach erfolgter Reinigung reduziert beziehungsweise ausgeschlossen werden.

Die Reinigung von Gewebearealen erfolgt erfindungsgemäß mechanisch durch Einwirkung der elastisch verformbaren Borsten und gleichzeitig durch ein oder mehrere nicht gepulste oder gepulste Spülflüssigkeitsstrahlen, wobei die Spülflüssigkeit von außen durch eine Schlauchzuleitung in die Vorrichtung geleitet werden kann. Die Spülflüssigkeit wird dabei erfindungsgemäß durch die in der chirurgischen Reinigungsvorrichtung enthaltenen Pumpe angesaugt und anschließend in Form von Sprühstrahlen wieder ausgestoßen. Diese Spülflüssigkeitsstrahlen strömen entweder ungepulst oder gepulst aus der chirurgischen Reinigungsvorrichtung aus. Weiterhin ist es möglich, die chirurgische Reinigungsvorrichtung mit Hilfe einer Absaugeinrichtung derart auszugestalten, dass die gebrauchte Spülflüssigkeit nach erfolgter Reinigung mit Hilfe der chirurgischen Reinigungsvorrichtung aus dem gereinigten Gewebeareal zumindest teilweise wieder entfernt werden kann.

Die gleichzeitige Anwendung von sich drehenden elastisch verformbaren Borsten und zumindest eines Sprühstrahls einer Spülflüssigkeit und der erfindungsgemäße Aufbau der chirurgischen Reinigungsvorrichtung ermöglichen, dass synchron zum Antrieb der rotierenden elastisch verformbaren Borsten gleichzeitig Spülflüssigkeit ausgesprüht werden kann und gegebenenfalls auch die gebrauchte Spülflüssigkeit wieder angesaugt werden kann, so dass ein Reinigungseffekt durch die gleichzeitige Einwirkung der rotierenden elastisch verformbaren Borsten zusammen mit Spülflüssigkeitsstrahlen erfolgen kann. Durch die bewegten elastisch verformbaren Borsten mechanisch abgetragene Verunreinigungen werden durch die gleichzeitig einwirkenden Spülflüssigkeitsstrahlen weggespült. Dadurch wird ein erneutes Anhaften der Verunreinigungen an den gereinigten Oberflächen verhindert.

Elektrische Antriebseinheiten für chirurgische Werkzeuge sind weltweit in der Chirurgie und Orthopädie üblich. Diese werden zum Antrieb von oszillierenden Sägen und zum Bohren genutzt. Als Energiequelle für solche Antriebseinheiten werden üblicherweise Akkumulatoren eingesetzt. Die erfindungsgemäße chirurgische Reinigungsvorrichtung kann problemlos mit dem hinteren Ende der Antriebswelle über ein Bohrfutter mit der externen Antriebseinheit verbunden werden. Der medizinische Anwender hält mit einer Hand die Antriebseinheit und betätigt mit der anderen Hand die Antriebsvorrichtung. Durch das Festhalten der chirurgischen Reinigungsvorrichtung wird ein Mitdrehen der chirurgischen Reinigungsvorrichtung mit der Antriebswelle verhindert. Bei der Verwendung einer Antriebswellenverlängerung kann die externe Antriebseinheit auch einfach an einem entfernten Ort aufgestellt und fixiert werden und der Anwender muss nur die chirurgische Reinigungsvorrichtung halten.

Eine beispielhafte erfindungsgemäße chirurgische Reinigungsvorrichtung ist zusammengesetzt aus
a) einem als Gehäuse ausgebildeten Griffstück (Handgriff),
b) einer frei drehbaren Antriebswelle, die bereichsweise im Gehäuse angeordnet ist,
c) einem aus dem Gehäuse herausragenden hinteren Wellenende der Antriebswelle, das für die Verbindung mit einer Antriebseinheit bestimmt ist,
d) einem vorderen Wellenende der Antriebswelle, das aus dem Gehäuse herausragt und an dem elastisch verformbare Borsten angebracht sind,
e) einem Wellengehäuse, das mit dem Griffstück verbunden ist, wobei das Wellengehäuse zu mindestens bereichsweise die Antriebswelle umgibt und in dem das vordere Wellenende gelagert ist, wobei die elastisch verformbaren Borsten des vorderen Wellenendes mindestens teilweise aus dem Wellengehäuse herausragen,
f) einer Pumpe, die mit der Antriebswelle verbunden ist,
g) einer Flüssigkeitszuleitung, die mit der Pumpe verbunden ist,
h) einer von der Pumpe ausgehenden Flüssigkeitsableitung, die eine oder mehrere Flüssigkeitsaustrittsöffnungen besitzt, die auf den Bereich der elastisch verformbaren Borsten ausgerichtet sind,
i) wobei der Antrieb der chirurgischen Reinigungsvorrichtung durch eine externe Antriebseinheit erfolgt, die mit dem hinteren Wellenende der chirurgischen Reinigungsvorrichtung verbunden ist oder verbindbar ist, und
j) wobei bei der Drehbewegung der Antriebswelle die elastisch verformbaren Borsten am vorderen Wellenende um eine Längsachse der Antriebswelle rotieren und die Pumpe gleichzeitig durch die Drehbewegung der Antriebswelle mit angetrieben wird.

Ein beispielhaftes erfindungsgemäßes Verfahren zur Reinigung mit einer erfindungsgemäßen chirurgischen Reinigungsvorrichtung kann dadurch charakterisiert sein, dass die Reinigung durch um eine Längsachse einer Antriebswelle rotierende elastisch verformbare Borsten erfolgt, wobei gleichzeitig mit der Drehbewegung der elastisch verformbaren Borsten, ungepulste oder gepulste Spülflüssigkeitsstrahlen in den Bereich der elastisch verformbaren Borsten gespritzt werden, und wobei der Antrieb der rotierenden elastisch verformbaren Borsten und der Pumpantrieb der Spülflüssigkeit durch eine mit der chirurgischen Reinigungsvorrichtung verbundene externe Antriebseinheit erfolgt.

Im Folgenden werden weitere Ausführungsbeispiele der Erfindung anhand von siebzehn schematisch dargestellten Figuren erläutert, ohne jedoch dabei die Erfindung zu beschränken. Dabei zeigt:
Figur 1: eine schematische Seitenansicht einer beispielhaften ersten erfindungsgemäßen chirurgischen Reinigungsvorrichtung;
Figur 2: eine schematische perspektivische Ansicht der ersten erfindungsgemäßen chirurgischen Reinigungsvorrichtung nach Figur 1 mit geschnittenem Gehäuse;
Figur 3: eine schematische perspektivische Teilquerschnittansicht der ersten erfindungsgemäßen chirurgischen Reinigungsvorrichtung nach den Figuren 1 und 2 zur Veranschaulichung des inneren Aufbaus;
Figur 4: eine schematische perspektivische Ausschnittvergrößerung als Detailansicht auf den vorderen Teil der ersten erfindungsgemäßen chirurgischen Reinigungsvorrichtung nach den Figuren 1 bis 3;
Figur 5: zwei übereinander dargestellte Detail-Querschnittansichten des vorderen Teils der ersten erfindungsgemäßen chirurgischen Reinigungsvorrichtung nach Figur 4;
Figur 6: eine schematische Querschnittssicht einer Impellerpumpe für eine erfindungsgemäße chirurgische Reinigungsvorrichtung;
Figur 7: eine schematische perspektivische Teilquerschnittssicht der Impellerpumpe nach Figur 6;
Figur 8: eine schematische perspektivische Ansicht der zweiten erfindungsgemäßen chirurgischen Reinigungsvorrichtung (Mitte) mit zwei Ausschnittvergrößerungen in der gleichen Perspektive (links oben und rechts unten);
Figur 9: eine schematische Teilquerschnittssicht einer dritten erfindungsgemäßen chirurgischen Reinigungsvorrichtung;
Figur 10: eine schematische Querschnittssicht einer Ausschnittvergrößerung der dritten erfindungsgemäßen chirurgischen Reinigungsvorrichtung nach Figur 9 beim Ausstoßen von Spülflüssigkeit bei geöffneten Flüssigkeitsaustrittsöffnungen;
Figur 11: eine schematische Querschnittssicht einer Ausschnittvergrößerung der dritten erfindungsgemäßen chirurgischen Reinigungsvorrichtung nach Figur 9 mit geschlossenen Flüssigkeitsaustrittsöffnungen;
Figur 12: eine schematische perspektivische Ansicht einer ersten Absaugeinrichtung als Aufsatz einer erfindungsgemäßen chirurgischen Reinigungsvorrichtung;
Figur 13: eine schematische perspektivische Ansicht einer zweiten Absaugeinrichtung als Aufsatz einer erfindungsgemäßen chirurgischen Reinigungsvorrichtung;
Figur 14: fünf schematische perspektivische Ansichten auf unterschiedliche erfindungsgemäße chirurgische Reinigungsvorrichtungen;
Figur 15: drei schematische perspektivische Detailansichten der zweiten erfindungsgemäßen chirurgischen Reinigungsvorrichtung mit aufgesetzter zweiter Absaugeinrichtung;
Figur 16: eine schematische perspektivische Ansicht (links oben), eine schematische perspektivische Querschnittansicht (Mitte) und eine schematische Querschnittansicht (rechts unten) auf eine Antriebswellenverlängerung für eine erfindungsgemäße chirurgische Reinigungsvorrichtung; und
Figur 17: zwei schematische perspektivische Teilansichten auf die Rückseite der dritten erfindungsgemäßen chirurgischen Reinigungsvorrichtung mit angeschlossener Antriebswellenverlängerung (links unten) und mit getrennter Antriebswellenverlängerung (rechts oben).

In den Figuren und in der folgenden Beschreibung zu den anhand der Figuren erläuterten Ausführungsbeispielen der vorliegenden Erfindung werden teilweise für unterschiedliche Ausführungsbeispiele und für unterschiedliche Einzelteile die gleichen Bezugszeichen bei gleichen oder gleichartigen Teilen verwendet, um die Vergleichbarkeit der Ausführungsbeispiele und die Lesbarkeit zu vereinfachen.

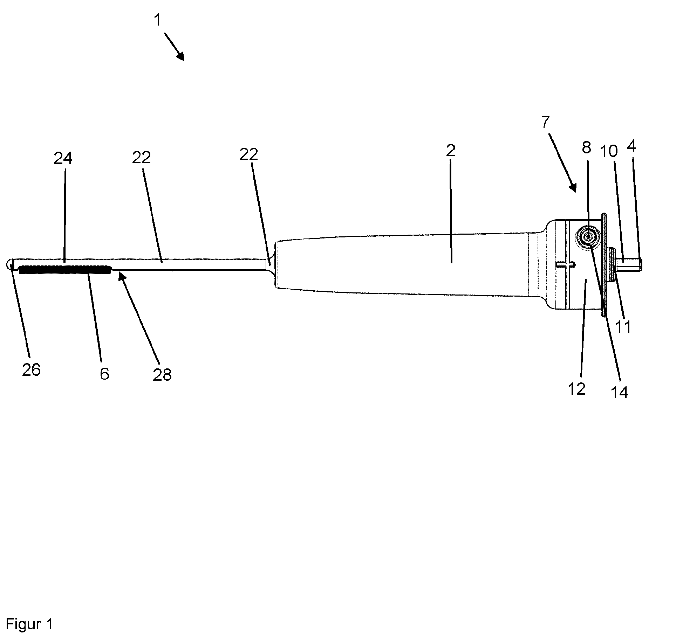

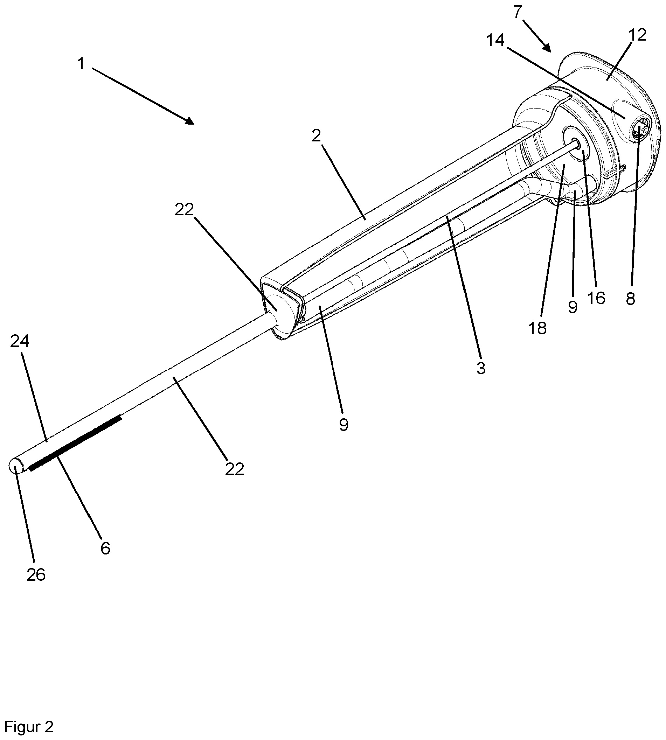

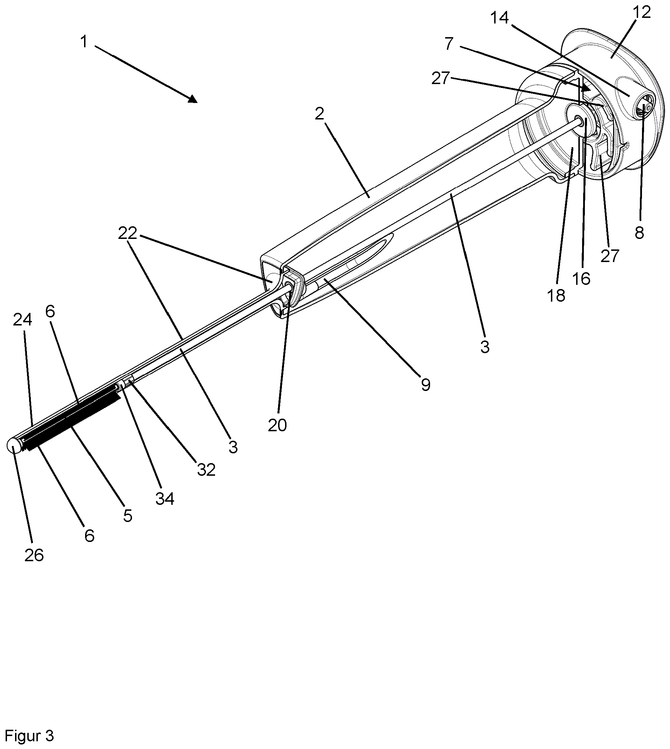

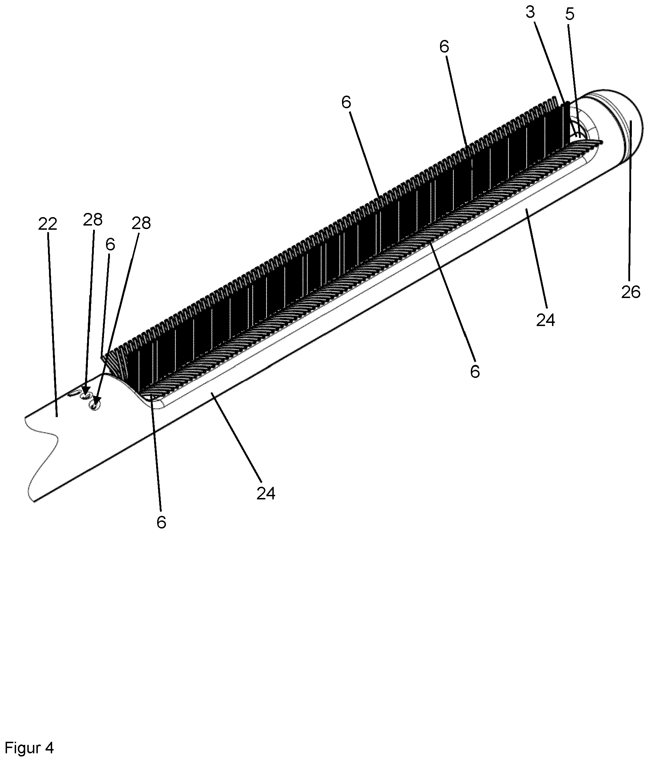

Figur 1 zeigt eine schematische Querschnittansicht einer beispielhaften ersten erfindungsgemäßen chirurgischen Reinigungsvorrichtung 1. In den Figuren 2 bis 7 sind weitere Ansichten, Querschnittansichten und Ausschnittvergrößerungen der chirurgischen Reinigungsvorrichtung 1 und ihrer Teile gezeigt. Die chirurgische Reinigungsvorrichtung 1 kann ein Gehäuse 2 aufweisen, das die chirurgische Reinigungsvorrichtung 1 nach außen abschließt und in einem hinteren Bereich als Griffstück zum Halten der chirurgischen Reinigungsvorrichtung 1 verwendet werden kann.

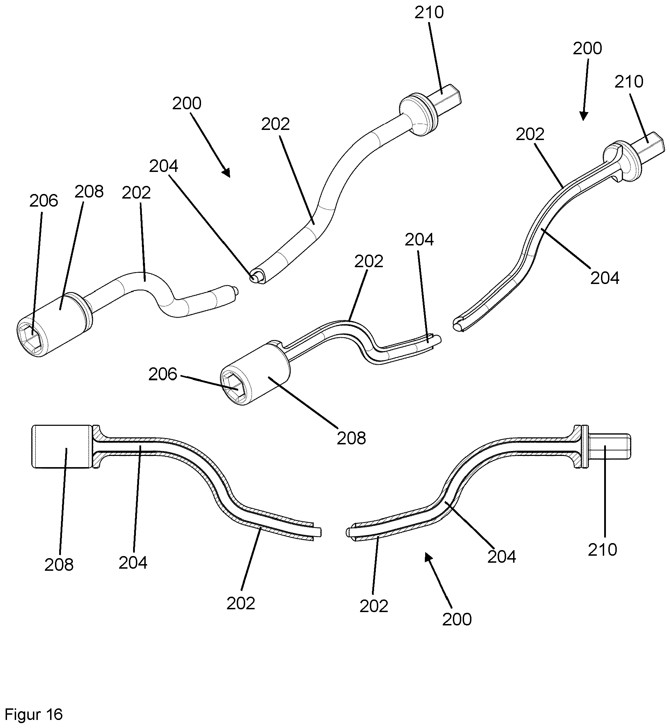

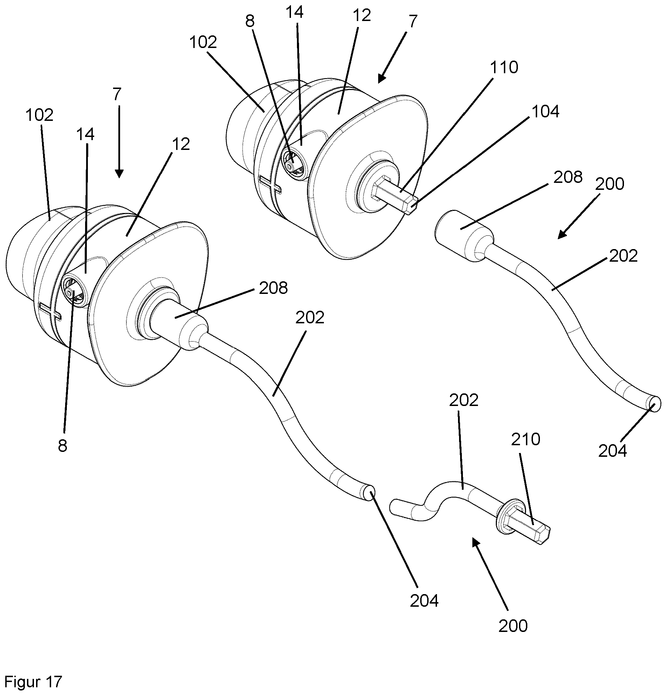

Im Inneren des Gehäuses 2 befindet sich eine darin drehbar gelagerte Antriebswelle 3. Die Antriebswelle 3 weist ein hinteres Ende 4 auf, das direkt oder über eine Antriebswellenverlängerung 200 (siehe Figuren 16 und 17) an eine externe Antriebseinheit (nicht gezeigt) angeschlossen oder anschließbar ist. Die externe Antriebseinheit kann beispielsweise ein im OP-Bereich üblicher Mehrzweck-Antrieb sein oder eine Bohrmaschine oder ähnliches. Dem hinteren Ende 4 gegenüberliegend weist die Antriebswelle 3 ein vorderes Ende 5 auf, an dem eine Vielzahl elastisch verformbarer Borsten 6 befestigt sind. Die elastisch verformbaren Borsten 6 können radial von der Antriebswelle 3 abstehen und bilden mit dem vorderen Ende 5 der Antriebswelle 3 eine gegen das Gehäuse 2 drehbare gelagerte Bürste.

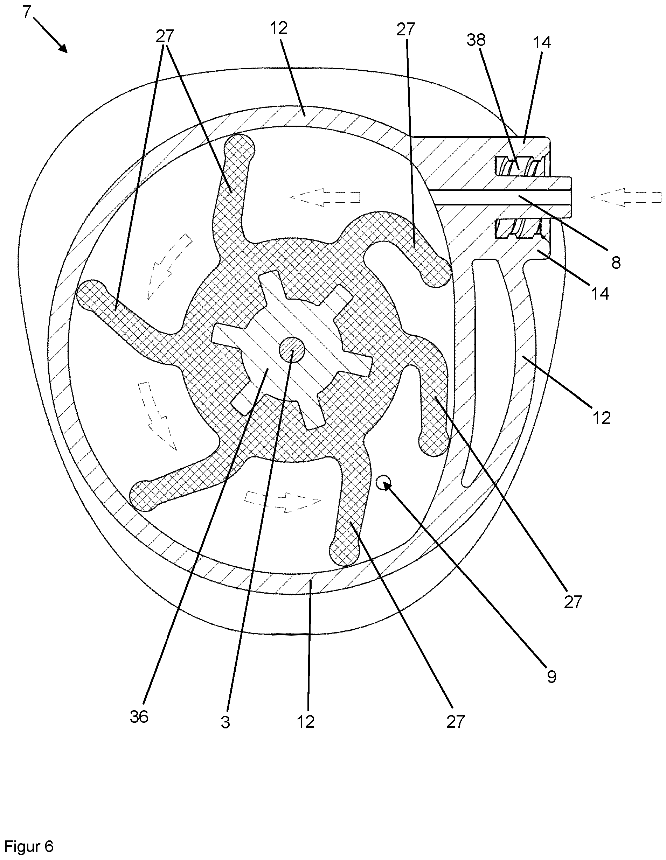

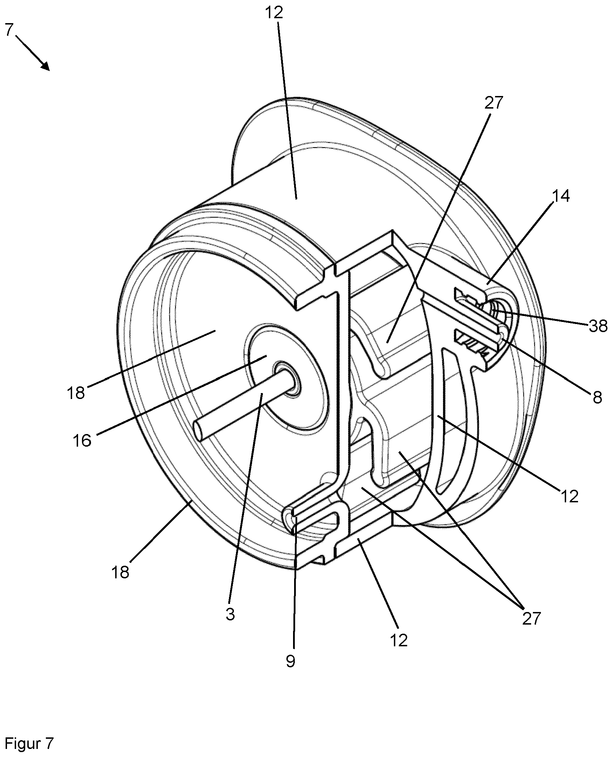

Die chirurgische Reinigungsvorrichtung 1 weist ferner eine Pumpe 7 auf, die mit der Antriebswelle 3 im Bereich des hinteren Endes 4 der Antriebswelle 3 verbunden sein kann. Die Pumpe 7 kann über die Antriebswelle 3 angetrieben werden und kann zu diesem Zweck mit der Antriebswelle 3 verbunden sein. Die Pumpe 7 kann bevorzugt als Impellerpumpe ausgeführt sein und ist in den Figuren 6 und 7 im Detail gezeigt. Die Pumpe 7 ist an eine Flüssigkeitszuleitung 8 zum Zuführen einer Spülflüssigkeit in die Pumpe 7 und an eine Flüssigkeitsableitung 9 zum Ableiten der Spülflüssigkeit aus der Pumpe 7 angeschlossen. Anstatt der Impellerpumpe kann auch eine andere Pumpe verwendet werden. Die Impellerpumpe ist jedoch aufgrund ihres einfachen Aufbaus und aufgrund der Tatsache, dass die Impellerpumpe bei einem Stillstand der Impellerpumpe die Flüssigkeitszuleitung 8 von der Flüssigkeitsableitung 9 physikalisch trennt, besonders bevorzugt.

Die Antriebswelle 3 kann am hinteren Ende 4 als Anschluss 10 in Form eines Sechskants ausgebildet sein. An den Anschluss 10 kann ein Spannfutter oder ein passender Gegenanschluss der externen Antriebseinrichtung angeschlossen sein oder werden. Die Antriebswellenverlängerung 200 kann zwischen den Anschluss 10 und den Gegenanschluss der externen Antriebseinrichtung angeschlossen werden. Der Anschluss 10 kann über ein kreisscheibenförmiges Gegenlager 11 in einem Pumpengehäuse 12 der Pumpe 7 gelagert sein.

Am Pumpengehäuse 12 kann ein Anschluss 14 zum Verbinden der Flüssigkeitszuleitung 8 mit einer Zuleitung von einer Spülflüssigkeitsquelle (nicht gezeigt) ausgebildet sein. Das Pumpengehäuse 12 kann auf seiner Vorderseite mit einem Pumpendeckel 18 verschlossen sein. Die Antriebswelle 3 kann über ein Lager 16 in die Pumpe 7 geführt sein.

Das Gehäuse 2 kann einen hinteren dickeren Bereich aufweisen, der als Griffstück fungiert, und ein Wellengehäuse 22 als vorderen schlankeren Bereich aufweisen. Das Griffstück des Gehäuses 2 kann an seiner Vorderseite ein scheibenförmiges Lager 20 aufweisen. In dem scheibenförmigen Lager 20 kann eine Durchführung angeordnet sein, in die die Flüssigkeitsableitung 9 in dem Griffstück des Gehäuses 2 mündet. Von dort aus kann die Flüssigkeitsableitung 9 durch das Wellengehäuse 22 bis zu mehreren Flüssigkeitsaustrittsöffnungen 28 geführt sein. Die Flüssigkeitsaustrittsöffnungen 28 münden im Bereich der elastische verformbaren Borsten 6 in deren Wirkbereich, das heißt in den Bereich, den die elastisch verformbaren Borsten 6 bei einer Drehung der Antriebswelle 3 überstreichen.

Das Wellengehäuse 22 kann an seiner Vorderseite als ein Spritzschutz 24 ausgeformt sein, der die elastisch verformbaren Borsten 6 bereichsweise umschließt. An der Vorderseite kann eine Kappe 26 angeordnet sein, die Antriebswelle 3 an ihrer vorderen Spitze abdeckt.

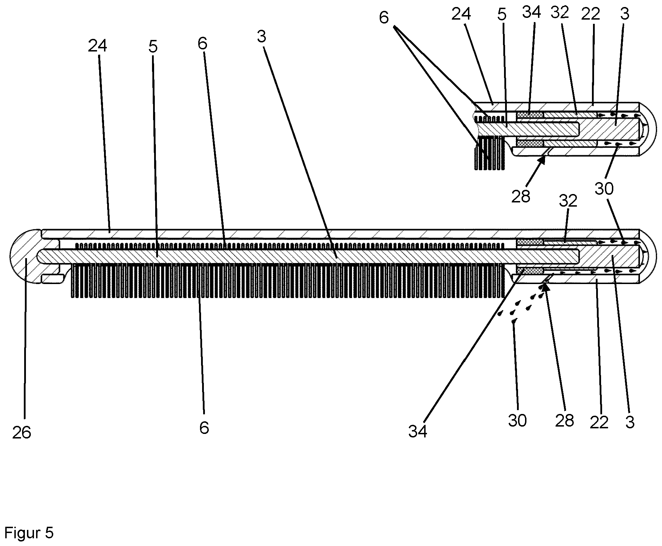

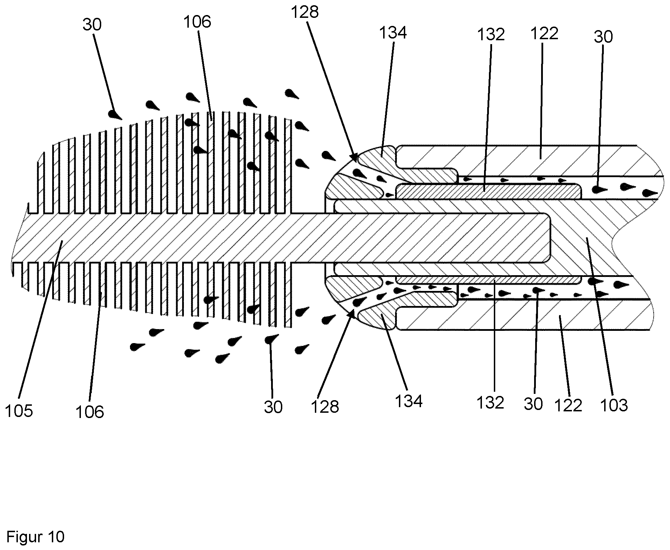

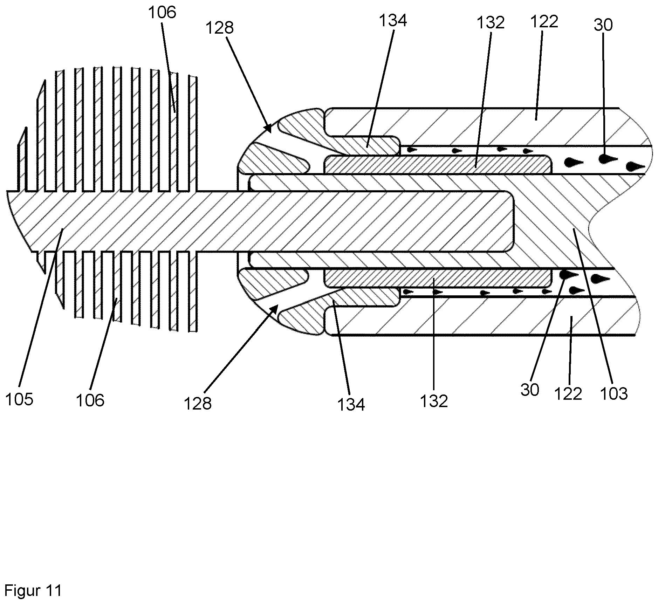

In der Pumpe 7 kann ein Impeller mit mehreren Impellerflügeln 27 angeordnet sein. Der Impeller kann auf der Antriebswelle 3 befestigt sein und sich mit der Antriebswelle 3 drehen. Hierzu kann eine Aufnahme 36 auf der Antriebswelle 3 befestigt sein, die mit dem Impeller kraftschlüssig verbunden ist. Durch die Drehung des Impellers mit der Antriebswelle 3 kann dann die Pumpe 7 betrieben werden. Auf diese Weise erfolgt gleichzeitig mit dem Drehen der elastisch verformbaren Borsten 6 das Pumpen der Spülflüssigkeit 30 mit der Pumpe 7 und damit das Ausstoßen eines Flüssigkeitsstrahls der Spülflüssigkeit 30 in den Wirkbereich der elastisch verformbaren Borsten 6 (siehe Figur 5 und analog den Figuren 10 und 11).

Um einen pulsierenden Strom der Spülflüssigkeit 30 zu erzeugen, kann in dem durch das Wellengehäuse 20 gebildeten Teilbereich der Spülflüssigkeitsableitung 9 eine Blende 32 auf der Antriebswelle 3 angeordnet sein. Die Blende 32 kann Bereiche mit unterschiedlich großen Radien aufweisen, so dass bei einer vollen Drehung der Antriebswelle 3 die Flüssigkeitsaustrittsöffnungen 28 beziehungsweise die Flüssigkeitsableitung 9 davor zeitweise geöffnet sind (siehe Figur 5 unten) und zeitweise verschlossen sind (siehe Figur 5 oben). Hierdurch wird ein pulsierender Ausstoß der Spülflüssigkeit 30 erzeugt, der mit der Umdrehungsgeschwindigkeit der Antriebswelle 3 getaktet ist. Die Antriebswelle 3 kann über eine Dichtung 34 durch die Vorderseite des Wellengehäuses 22 geführt sein. Gleichzeitig mit dem Antrieb der Drehung der elastisch verformbaren Borsten 6 und dem Ausstoß der Spülflüssigkeit 30 kann also auch ein pulsierender Betrieb des Spülflüssigkeitsstroms über die Drehung der Antriebswelle 3 erzeugt werden.

In dem Anschluss 14 für die Flüssigkeitszuleitung 8 kann ein Innengewinde 38 zur Befestigung einer Zuleitung für die Spülflüssigkeit 30 angeordnet sein. Die Pumpe 7 kann dann mit dem Anschluss 14 mit der Spülflüssigkeitsquelle (nicht gezeigt) verbunden werden. Die chirurgische Reinigungsvorrichtung 1 kann betrieben werden, indem die chirurgische Reinigungsvorrichtung 1 mit dem Anschluss 10 der Antriebswelle 3 direkt oder über eine Antriebswellenverlängerung 200 mit der externen Antriebseinheit verbunden wird. Die externe Antriebseinheit treibt die Antriebswelle 3 an. Durch die Drehung der Antriebswelle 3 wird die Pumpe 7 betrieben und gleichzeitig die mit den elastisch verformbaren Borsten 6 aufgebaute Bürste gedreht. Die Pumpe 7 erzeugt einen Strom der Spülflüssigkeit 30, der über die Spülflüssigkeitsaustrittsöffnungen 28 in Richtung der elastisch verformbaren Borsten 6 ausgestoßen wird. Mit Hilfe der Blende 32 kann ein pulsierender Strom der Spülflüssigkeit 30 erzeugt werden. Mit der chirurgischen Reinigungsvorrichtung 1 kann so eine mechanische Reinigung bei gleichzeitiger Einwirkung des Flüssigkeitsstrahls der Spülflüssigkeit 30 erfolgen.

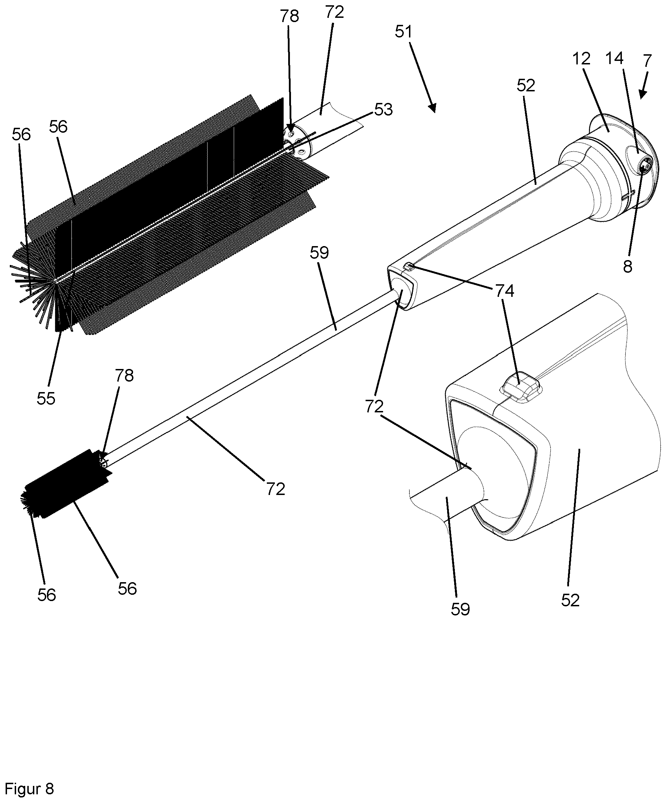

Figur 8 zeigt eine schematische Querschnittansicht einer beispielhaften zweiten erfindungsgemäßen chirurgischen Reinigungsvorrichtung 51. Die chirurgische Reinigungsvorrichtung 51 kann ein Gehäuse 52 aufweisen, das die chirurgische Reinigungsvorrichtung 51 nach außen abschließt und in einem hinteren Bereich als Griffstück zum Halten der chirurgischen Reinigungsvorrichtung 51 verwendet werden kann. Der innere Aufbau der chirurgischen Reinigungsvorrichtung 51 gleicht dem des ersten Ausführungsbeispiels.

Im Inneren des Gehäuses 52 befindet sich eine darin drehbar gelagerte Antriebswelle 53 (in Figur 8 nicht im Inneren des Gehäuses 52 zu sehen aber analog der Antriebswelle 3 nach den Figuren 2, 3 und 5). Die Antriebswelle 53 weist ein hinteres Ende auf (in Figur 8 nicht zu sehen aber analog dem ersten Ausführungsbeispiel nach Figur 1), wobei das hintere Ende direkt oder über eine Antriebswellenverlängerung 200 (siehe Figuren 16 und 17) an eine externe Antriebseinheit (nicht gezeigt) angeschlossen oder anschließbar ist. Die externe Antriebseinheit kann beispielsweise ein im OP-Bereich üblicher Mehrzweck-Antrieb sein oder eine Bohrmaschine oder ähnliches. Dem hinteren Ende gegenüberliegend weist die Antriebswelle 53 ein vorderes Ende 55 auf, an dem eine Vielzahl elastisch verformbarer Borsten 56 befestigt sind. Das vordere Ende 55 der Antriebswelle 53 mit den Borsten 56 ragt aus dem Gehäuse 52 heraus. Die elastisch verformbaren Borsten 56 können radial von der Antriebswelle 53 und auch axial von der Spitze des vorderen Endes 55 der Antriebswelle 53 abstehen und bilden mit dem vorderen Ende 55 der Antriebswelle 53 eine gegen das Gehäuse 52 drehbare gelagerte Bürste.

Die chirurgische Reinigungsvorrichtung 51 weist ferner eine Pumpe 7 auf, die mit der Antriebswelle 53 im Bereich des hinteren Endes der Antriebswelle 53 verbunden sein kann. Die Pumpe 7 kann über die Antriebswelle 53 angetrieben werden und kann zu diesem Zweck mit der Antriebswelle 53 verbunden sein. Die Pumpe 7 kann bevorzugt als Impellerpumpe ausgeführt sein und ist in den Figuren 6 und 7 im Detail gezeigt. Die Pumpe 7 ist an eine Flüssigkeitszuleitung 8 zum Zuführen einer Spülflüssigkeit in die Pumpe 7 und an eine Flüssigkeitsableitung 59 zum Ableiten der Spülflüssigkeit aus der Pumpe 7 angeschlossen. Der Teil der Flüssigkeitsableitung 59, der in dem das Griffstück bildenden Teil des Gehäuses 52 analog der Flüssigkeitsableitung 9 nach dem ersten Ausführungsbeispiel (siehe Figur 2) ausgebildet ist, ist in Figur 8 nicht zu sehen. Anstatt der Impellerpumpe kann auch eine andere Pumpe verwendet werden. Die Impellerpumpe ist jedoch aufgrund ihres einfachen Aufbaus und aufgrund der Tatsache, dass die Impellerpumpe bei einem Stillstand der Impellerpumpe die Flüssigkeitszuleitung 8 von der Flüssigkeitsableitung 59 physikalisch trennt, besonders bevorzugt.

Die Antriebswelle 53 kann am hinteren Ende einen Anschluss aufweisen. An den Anschluss kann ein Spannfutter oder ein passender Gegenanschluss der externen Antriebseinrichtung angeschlossen sein oder werden. Die Antriebswellenverlängerung 200 kann zwischen den Anschluss und den Gegenanschluss der externen Antriebseinrichtung angeschlossen werden.

Die Pumpe 7 kann ein Pumpengehäuse 12 aufweisen, an dem ein Anschluss 14 zum Verbinden der Flüssigkeitszuleitung 8 mit einer Zuleitung von einer Spülflüssigkeitsquelle (nicht gezeigt) ausgebildet ist. Das Pumpengehäuse 12 kann auf seiner Vorderseite mit einem Pumpendeckel 18 verschlossen sein. Die Antriebswelle 53 kann über ein Lager 16 in die Pumpe 7 geführt sein.

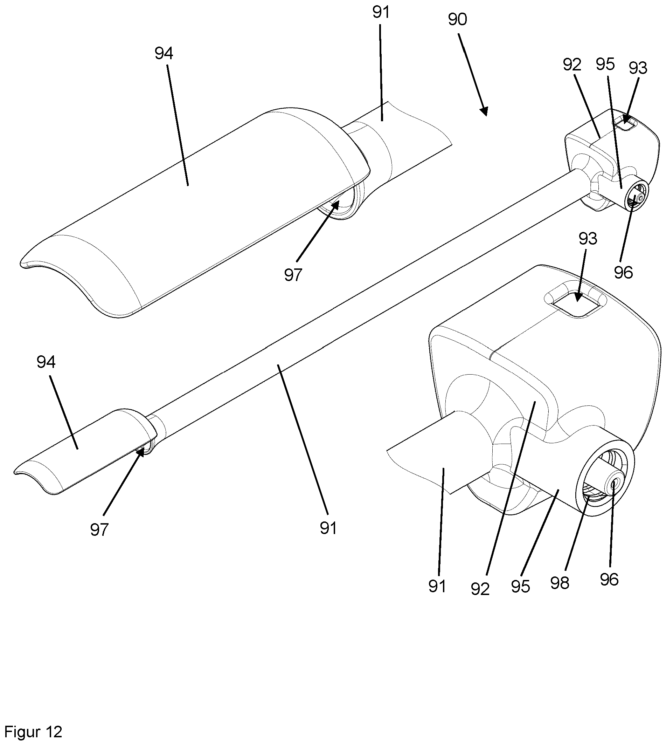

Das Gehäuse 52 kann einen hinteren dickeren Bereich aufweisen, der als Griffstück fungiert, und ein Wellengehäuse 72 als vorderen schlankeren Bereich aufweisen. Das Griffstück des Gehäuses 52 kann an seiner Vorderseite ein scheibenförmiges Lager aufweisen. An der Oberseite des Griffstücks des Gehäuses 52 kann ein Vorsprung 74 zur Befestigung einer Absaugeinrichtung 90, 140 angeordnet sein (siehe die Figuren 12 bis 15). In dem scheibenförmigen Lager kann eine Durchführung angeordnet sein, in die der Teil der Flüssigkeitsableitung 59 in dem Griffstück des Gehäuses 2 mündet. Von dort aus kann die Flüssigkeitsableitung 59 durch das Wellengehäuse 22 bis zu mehreren Flüssigkeitsaustrittsöffnungen 78 geführt sein. Die Flüssigkeitsaustrittsöffnungen 78 münden im Bereich der elastische verformbaren Borsten 56 in deren Wirkbereich, das heißt in den Bereich, den die elastisch verformbaren Borsten 56 bei einer Drehung der Antriebswelle 53 überstreichen.

In der Pumpe 7 kann ein Impeller mit mehreren Impellerflügeln 27 angeordnet sein. Der Impeller kann auf der Antriebswelle 53 befestigt sein und sich mit der Antriebswelle 53 drehen. Hierzu kann eine Aufnahme 36 auf der Antriebswelle 53 befestigt sein, die mit dem Impeller kraftschlüssig verbunden ist. Durch die Drehung des Impellers mit der Antriebswelle 53 kann dann die Pumpe 7 betrieben werden. Auf diese Weise erfolgt gleichzeitig mit dem Drehen der elastisch verformbaren Borsten 56 das Pumpen der Spülflüssigkeit mit der Pumpe 7 und damit das Ausstoßen eines Flüssigkeitsstrahls der Spülflüssigkeit in den Wirkbereich der elastisch verformbaren Borsten 56.

Die Pumpe 7 kann mit dem Anschluss 14 mit einer Spülflüssigkeitsquelle (nicht gezeigt) verbunden werden. Die chirurgische Reinigungsvorrichtung 51 kann betrieben werden, indem die chirurgische Reinigungsvorrichtung 51 mit dem Anschluss der Antriebswelle 53 direkt oder über eine Antriebswellenverlängerung 200 mit der externen Antriebseinheit verbunden wird.

Die externe Antriebseinheit treibt die Antriebswelle 53 an. Durch die Drehung der Antriebswelle 53 wird die Pumpe 7 betrieben und gleichzeitig die mit den elastisch verformbaren Borsten 56 aufgebaute Bürste gedreht. Die Pumpe 7 erzeugt einen Strom der Spülflüssigkeit, der über die Spülflüssigkeitsaustrittsöffnungen 78 in Richtung der elastisch verformbaren Borsten 56 ausgestoßen wird. Mit der chirurgischen Reinigungsvorrichtung 51 kann so eine mechanische Reinigung bei gleichzeitiger Einwirkung des Flüssigkeitsstrahls der Spülflüssigkeit erfolgen.

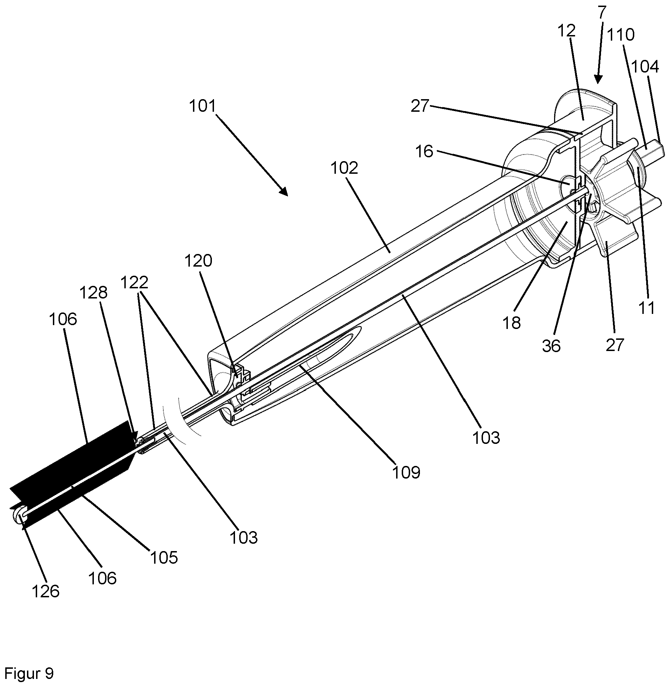

Figur 9 zeigt eine schematische Querschnittansicht einer beispielhaften dritten erfindungsgemäßen chirurgischen Reinigungsvorrichtung 101. In den Figuren 10 und 11 sind Querschnittansichten als Ausschnittvergrößerungen der chirurgischen Reinigungsvorrichtung 101 gezeigt. Die chirurgische Reinigungsvorrichtung 101 kann ein Gehäuse 102 aufweisen, das die chirurgische Reinigungsvorrichtung 101 nach außen abschließt und in einem hinteren Bereich als Griffstück zum Halten der chirurgischen Reinigungsvorrichtung 101 verwendet werden kann.

Im Inneren des Gehäuses 102 befindet sich eine darin drehbar gelagerte Antriebswelle 103. Die Antriebswelle 103 weist ein hinteres Ende 104 auf, das direkt oder über eine Antriebswellenverlängerung 200 (siehe Figuren 16 und 17) an eine externe Antriebseinheit (nicht gezeigt) angeschlossen oder anschließbar ist. Die externe Antriebseinheit kann beispielsweise ein im OP-Bereich üblicher Mehrzweck-Antrieb sein oder eine Bohrmaschine oder ähnliches. Dem hinteren Ende 104 gegenüberliegend weist die Antriebswelle 103 ein vorderes Ende 105 auf, an dem eine Vielzahl elastisch verformbarer Borsten 106 befestigt sind. Die elastisch verformbaren Borsten 106 können radial von der Antriebswelle 103 abstehen und bilden mit dem vorderen Ende 105 der Antriebswelle 103 eine gegen das Gehäuse 102 drehbare gelagerte Bürste.

Die chirurgische Reinigungsvorrichtung 101 weist ferner eine Pumpe 7 auf, die mit der Antriebswelle 103 im Bereich des hinteren Endes 104 der Antriebswelle 103 verbunden sein kann. Die Pumpe 7 kann über die Antriebswelle 103 angetrieben werden und kann zu diesem Zweck mit der Antriebswelle 103 verbunden sein. Die Pumpe 7 kann bevorzugt als Impellerpumpe ausgeführt sein und ist in den Figuren 6 und 7 im Detail gezeigt. Die Pumpe 7 ist an eine Flüssigkeitszuleitung 8 zum Zuführen einer Spülflüssigkeit in die Pumpe 7 und an eine Flüssigkeitsableitung 109 zum Ableiten der Spülflüssigkeit aus der Pumpe 7 angeschlossen. Anstatt der Impellerpumpe kann auch eine andere Pumpe verwendet werden. Die Impellerpumpe ist jedoch aufgrund ihres einfachen Aufbaus und aufgrund der Tatsache, dass die Impellerpumpe bei einem Stillstand der Impellerpumpe die Flüssigkeitszuleitung 8 von der Flüssigkeitsableitung 109 physikalisch trennt, besonders bevorzugt.

Die Antriebswelle 103 kann am hinteren Ende 104 als Anschluss 110 in Form eines Sechskants ausgebildet sein. An den Anschluss 110 kann ein Spannfutter oder ein passender Gegenanschluss der externen Antriebseinrichtung angeschlossen sein oder werden. Die Antriebswellenverlängerung 200 kann zwischen den Anschluss 110 und den Gegenanschluss der externen Antriebseinrichtung angeschlossen werden. Der Anschluss 110 kann über ein kreisscheibenförmiges Gegenlager 11 in einem Pumpengehäuse 12 der Pumpe 7 gelagert sein.

Am Pumpengehäuse 12 kann ein Anschluss 14 zum Verbinden der Flüssigkeitszuleitung 8 mit einer Zuleitung von einer Spülflüssigkeitsquelle (nicht gezeigt) ausgebildet sein. Das Pumpengehäuse 12 kann auf seiner Vorderseite mit einem Pumpendeckel 18 verschlossen sein. Die Antriebswelle 103 kann über ein Lager 16 in die Pumpe 7 geführt werden.

Das Gehäuse 102 kann einen hinteren dickeren Bereich aufweisen, der als Griffstück fungiert, und ein Wellengehäuse 122 als vorderen schlankeren Bereich aufweisen. Das Griffstück des Gehäuses 102 kann an seiner Vorderseite ein scheibenförmiges Lager 120 aufweisen. In dem scheibenförmigen Lager 120 kann eine Durchführung angeordnet sein, in die die Flüssigkeitsableitung 109 in dem Griffstück des Gehäuses 102 mündet. Von dort aus kann die Flüssigkeitsableitung 109 durch das Wellengehäuse 122 bis zu mehreren Flüssigkeitsaustrittsöffnungen 128 geführt sein. Die Flüssigkeitsaustrittsöffnungen 128 münden im Bereich der elastische verformbaren Borsten 106 in deren Wirkbereich, das heißt in den Bereich, den die elastisch verformbaren Borsten 106 bei einer Drehung der Antriebswelle 103 überstreichen. An der Vorderseite kann eine Kappe 126 angeordnet sein, die Antriebswelle 103 an ihrer vorderen Spitze abdeckt.

In der Pumpe 7 kann ein Impeller mit mehreren Impellerflügeln 27 angeordnet sein. Der Impeller kann auf der Antriebswelle 103 befestigt sein und sich mit der Antriebswelle 103 drehen. Hierzu kann eine Aufnahme 36 auf der Antriebswelle 103 befestigt sein, die mit dem Impeller kraftschlüssig verbunden ist. Durch die Drehung des Impellers mit der Antriebswelle 103 kann dann die Pumpe 7 betrieben werden. Auf diese Weise erfolgt gleichzeitig mit dem Drehen der elastisch verformbaren Borsten 106 das Pumpen der Spülflüssigkeit 30 mit der Pumpe 7 und damit das Ausstoßen eines Flüssigkeitsstrahls der Spülflüssigkeit 30 in den Wirkbereich der elastisch verformbaren Borsten 106 (siehe Figuren 10 und 11).

Um einen pulsierenden Strom der Spülflüssigkeit 30 zu erzeugen, kann in dem durch das Wellengehäuse 120 gebildeten Teilbereich der Spülflüssigkeitsableitung 109 eine Blende 132 auf der Antriebswelle 103 angeordnet sein. Die Blende 132 kann Bereiche mit unterschiedlich großen Radien aufweisen, so dass bei einer vollen Drehung der Antriebswelle 103 die Flüssigkeitsaustrittsöffnungen 128 beziehungsweise die Flüssigkeitsableitung 109 davor zeitweise geöffnet sind (siehe Figur 10) und zeitweise verschlossen sind (siehe Figur 11). Hierdurch wird ein pulsierender Ausstoß der Spülflüssigkeit 30 erzeugt, der mit der Umdrehungsgeschwindigkeit der Antriebswelle 103 getaktet ist. Die Antriebswelle 103 kann über einen Einsatz 134 durch die Vorderseite des Wellengehäuses 122 geführt sein. Die Flüssigkeitsaustrittsöffnungen 128 können in dem Einsatz 134 ausgeformt sein. Gleichzeitig mit dem Antrieb der Drehung der elastisch verformbaren Borsten 106 und dem Ausstoß der Spülflüssigkeit 30 kann also auch ein pulsierender Betrieb des Spülflüssigkeitsstroms über die Drehung der Antriebswelle 103 erzeugt werden.

In dem Anschluss 14 für die Flüssigkeitszuleitung 8 kann ein Innengewinde 38 zur Befestigung einer Zuleitung für die Spülflüssigkeit 30 angeordnet sein. Die Pumpe 7 kann dann mit dem Anschluss 14 mit der Spülflüssigkeitsquelle (nicht gezeigt) verbunden werden. Die chirurgische Reinigungsvorrichtung 101 kann betrieben werden, indem die chirurgische Reinigungsvorrichtung 101 mit dem Anschluss 110 direkt oder über eine Antriebswellenverlängerung 200 (siehe Figur 17) mit der externen Antriebseinheit verbunden wird. Die externe Antriebseinheit treibt die Antriebswelle 103 direkt oder über die Antriebswellenverlängerung 200 an. Durch die Drehung der Antriebswelle 103 wird die Pumpe 7 betrieben und gleichzeitig die mit den elastisch verformbaren Borsten 106 aufgebaute Bürste gedreht. Die Pumpe 7 erzeugt einen Strom der Spülflüssigkeit 30, der über die Spülflüssigkeitsaustrittsöffnungen 128 in Richtung der elastisch verformbaren Borsten 106 ausgestoßen wird. Mit Hilfe der Blende 132 kann ein pulsierender Strom der Spülflüssigkeit 30 erzeugt werden. Mit der chirurgischen Reinigungsvorrichtung 101 kann so eine mechanische Reinigung bei gleichzeitiger Einwirkung des Flüssigkeitsstrahls der Spülflüssigkeit 30 erfolgen.

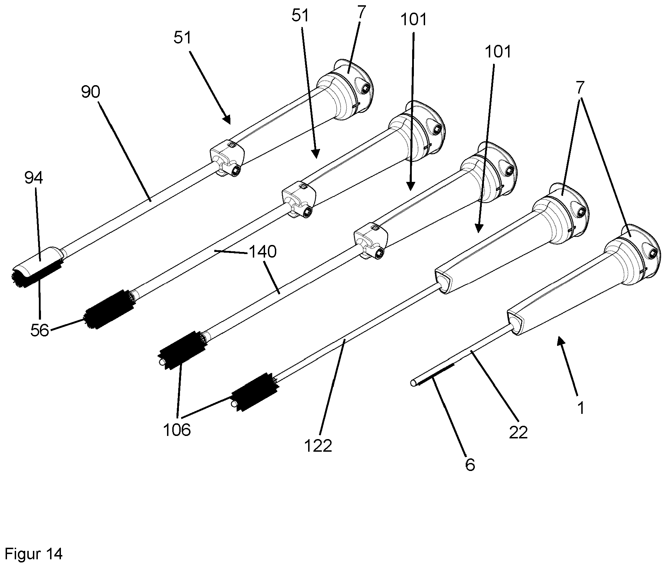

In Figur 12 ist eine erste Absaugeinrichtung 90 in einer schematischen perspektivischen Ansicht (Figur 12 Mitte) und in zwei schematischen perspektivischen Ausschnittvergrößerungen (Figur 12 links oben und rechts unten) gezeigt. Die Absaugeinrichtung 90 kann mit der zweiten oder dritten beispielhaften chirurgischen Reinigungsvorrichtung 51, 101 verwendet werden. Beispielhaft ist dies in der Figur 14 (Abbildung links oben) für die zweite beispielhafte chirurgische Reinigungsvorrichtung 51 gezeigt.

Die Absaugeinrichtung 90 kann eine Absaugleitung 91 zum Abführen gebrauchter Spülflüssigkeit aus dem Bereich der Bürste der chirurgischen Reinigungsvorrichtung 51, 101 aufweisen. An der hinteren Seite der Absaugeinrichtung 90 kann diese als Aufsteckbuchse 92 zur Verbindung mit dem Griffstück des Gehäuses 52, 102 einer der chirurgischen Reinigungsvorrichtungen 51, 101 ausgeformt sein. In der Aufsteckbuchse 92 kann eine Öffnung 93 zur Aufnahme des Vorsprungs 74 der chirurgischen Reinigungsvorrichtung 51 aufweisen (siehe Figur 14 links oben). Hierdurch wird eine stabilere Verbindung der Absaugeinrichtung 90 zu dem Gehäuse 52, 102 erreicht.

Die Absaugeinrichtung 90 kann an ihrer Vorderseite auch eine Spritzschutz 94 aufweisen, der die Borsten 56 der chirurgischen Reinigungsvorrichtung 51 bereichsweise abdeckt (siehe Figur 14 links oben). Im Bereich der Aufsteckbuchse 92 kann ein Ableitungsanschluss 95 mit einer Spülflüssigkeitsableitung 96 angeordnet sein. Die Spülflüssigkeitsableitung 96 ist mit der Absaugleitung 91 flüssigkeitsdurchlässig verbunden. Die Spülflüssigkeitsableitung 96 ist mit einer Unterdruckquelle (nicht gezeigt) verbunden oder verbindbar und dient dem Entfernen der gebrauchten Spülflüssigkeit. Derartige Unterdruckquellen sind in Krankenhäusern üblicherweise vorhanden. Zwischen der Unterdruckquelle und der Spülflüssigkeitsableitung 96 kann ein Abscheider (nicht gezeigt) zum Abtrennen fester Bestandteile aus der abgesaugten gebrauchten Spülflüssigkeit angeordnet sein.

An der Vorderseite der Absaugleitung 91 ist eine Absaugöffnung 97 vorgesehen, durch die die gebrauchte Spülflüssigkeit in der Absaugleitung 91 eingesaugt werden kann. Bevorzugt ist die Absaugöffnung 97 trichterförmig geformt. Zur dichten und festen Verbindung des Ableitungsanschlusses 95 mit der Unterdruckquelle kann ein Innengewinde 98 in dem Ableitungsanschluss 95 vorgesehen sein.

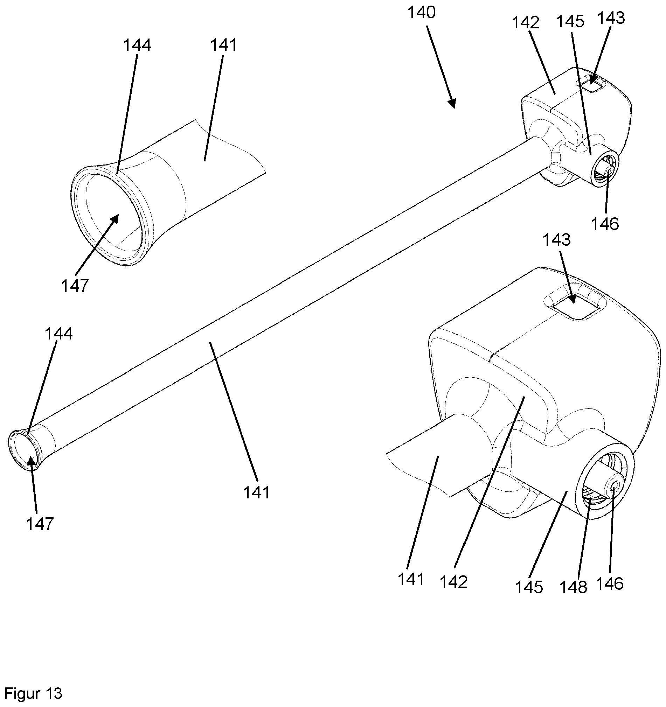

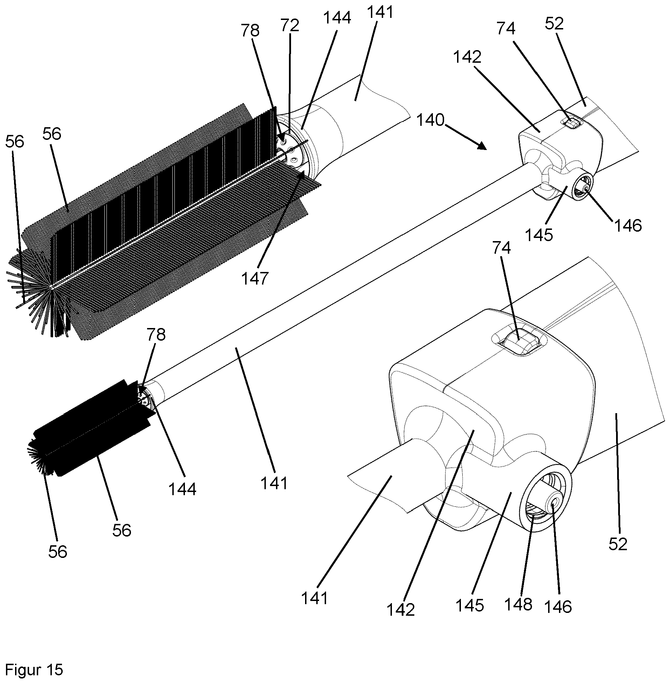

In Figur 13 ist eine zweite Absaugeinrichtung 140 in einer schematischen perspektivischen Ansicht (Mitte) und in zwei schematischen perspektivischen Ausschnittvergrößerungen (links oben und rechts unten) gezeigt. Als Teil einer erfindungsgemäßen chirurgischen Reinigungsvorrichtung ist die Absaugeinrichtung 140 in Figur 14 (zweites und drittes Bild von links oben) und in Figur 15 (als Ausschnittvergrößerung der zweiten Abbildung von links oben in Figur 14) gezeigt. Die Absaugeinrichtung 140 kann mit der zweiten oder der dritten beispielhaften chirurgischen Reinigungsvorrichtungen 51, 101 verwendet werden. Beispielhaft ist dies in den Figuren 15 und 14 (dort die zweite Abbildung von links oben) für die zweite beispielhafte chirurgische Reinigungsvorrichtung 51 sowie in Figur 14 (dort die mittlere Abbildung) für die dritte beispielhafte chirurgische Reinigungsvorrichtung 101 gezeigt.

Die Absaugeinrichtung 140 kann eine Absaugleitung 141 zum Abführen gebrauchter Spülflüssigkeit aus dem Bereich der Bürste der chirurgischen Reinigungsvorrichtung 51, 101 aufweisen. An der hinteren Seite der Absaugeinrichtung 140 kann diese als Aufsteckbuchse 142 zur Verbindung mit dem Griffstück des Gehäuses 52, 102 einer der chirurgischen Reinigungsvorrichtung 51, 101 ausgeformt sein. In der Aufsteckbuchse 142 kann eine Öffnung 143 zur Aufnahme des Vorsprungs 74 der chirurgischen Reinigungsvorrichtung 51 aufweisen (siehe Figur 14 zweite von links oben und Figur 15). Hierdurch wird eine stabilere Verbindung erreicht.

Im Bereich der Aufsteckbuchse 142 kann ein Ableitungsanschluss 145 mit einer Spülflüssigkeitsableitung 146 angeordnet sein. Die Spülflüssigkeitsableitung 146 ist mit der Absaugleitung 141 flüssigkeitsdurchlässig verbunden. Die Spülflüssigkeitsableitung 146 ist mit einer Unterdruckquelle (nicht gezeigt) verbunden oder verbindbar und dient dem Entfernen der gebrauchten Spülflüssigkeit. Derartige Unterdruckquellen sind in Krankenhäusern üblicherweise vorhanden. Zwischen der Unterdruckquelle und der Spülflüssigkeitsableitung 146 kann ein Abscheider (nicht gezeigt) zum Abtrennen fester Bestandteile aus der abgesaugten gebrauchten Spülflüssigkeit angeordnet sein.

An der Vorderseite der Absaugleitung 141 ist eine Absaugöffnung 147 vorgesehen, durch die die gebrauchte Spülflüssigkeit in der Absaugleitung 141 eingesaugt werden kann. Bevorzugt ist die Absaugöffnung 147 innerhalb eines Trichters 144 angeordnet. Zur dichten und festen Verbindung des Ableitungsanschlusses 145 mit der Unterdruckquelle kann ein Innengewinde 148 in dem Ableitungsanschluss 145 vorgesehen sein.

Es ist auch möglich, den Unterdruck, der an die Spülflüssigkeitsableitung 96, 146 einer der Absaugeinrichtungen 90, 140 angelegt wird, mit einer zweiten Pumpe (nicht gezeigt) zu erzeugen, die von der Antriebswelle 3, 53, 103 der chirurgischen Reinigungsvorrichtungen 1, 51, 101 angetrieben wird. Hierzu kann die zweite Pumpe mit der Antriebswelle 3, 53, 103 verbunden sein. Die zweite Pumpe kann Teil der erfindungsgemäßen Vorrichtung sein.

Figur 14 zeigt fünf verschiedene erfindungsgemäße chirurgische Reinigungsvorrichtungen als schematische perspektivische Außenansichten, die auf der ersten beispielhaften chirurgischen Reinigungsvorrichtung 1 nach den Figuren 1 bis 7, der zweiten beispielhaften chirurgischen Reinigungsvorrichtung 51 nach Figur 8 und der dritten beispielhaften chirurgischen Reinigungsvorrichtung 101 nach den Figuren 9 bis 11 basieren. Zur besseren Vergleichbarkeit, sind die chirurgischen Reinigungsvorrichtungen in einer gemeinsamen Figur 14 dargestellt. In Figur 14 links unten ist eine Abbildung der ersten beispielhaften chirurgischen Reinigungsvorrichtung 1 nach den Figuren 1 bis 7 gezeigt. In der Abbildung rechts darüber ist die dritte beispielhafte chirurgische Reinigungsvorrichtung 101 nach den Figuren 9 bis 11 gezeigt. In der mittleren Abbildung der Figur 14 ist eine chirurgische Reinigungsvorrichtung gezeigt, bei der die dritte beispielhafte chirurgische Reinigungsvorrichtung 101 nach den Figuren 9 bis 11 mit einer Absaugeinrichtung 140 nach Figur 13 kombiniert ist. In der zweiten Abbildung von links oben der Figur 14 ist eine chirurgische Reinigungsvorrichtung gezeigt, bei der die zweite beispielhafte chirurgische Reinigungsvorrichtung 51 nach Figur 8 mit einer Absaugeinrichtung 140 nach Figur 13 kombiniert ist. Figur 15 zeigt hierzu drei Ausschnittvergrößerungen dieser Ausführung. In der ersten Abbildung von links oben in Figur 14 ist eine chirurgische Reinigungsvorrichtung gezeigt, bei der die zweite beispielhafte chirurgische Reinigungsvorrichtung 51 nach Figur 8 mit einer Absaugeinrichtung 90 nach Figur 12 kombiniert ist.

Figur 16 zeigt eine schematische perspektivische Außenansicht (Oben), eine schematische perspektivische Teilschnittansicht (Mitte) und eine schematische Querschnittansicht (Unten) einer Antriebswellenverlängerung 200, die Teil einer erfindungsgemäßen chirurgische Reinigungsvorrichtung 1, 51, 101 sein kann oder über die eine erfindungsgemäße chirurgische Reinigungsvorrichtung 1, 51, 101 angetrieben werden kann, indem die Antriebswellenverlängerung 200 den Anschluss 10, 110 der Antriebswelle 3, 53, 103 mit einer externen Antriebseinrichtung verbindet.

Die Antriebswellenverlängerung 200 kann einen beweglichen Schlauch 202 aufweisen, in dem eine bewegliche Welle 204, wie beispielsweise ein Seil, angeordnet sein kann. Der bewegliche Schlauch 202 und die bewegliche Welle 204 darin können vorzugsweise nach Art eines Schlauchs gebogen und/oder gekrümmt werden. Zur Verbindung mit dem Anschluss 10, 110 der Antriebswelle 3, 53, 103 kann ein passendes Kantenloch 206 vorgesehen sein, in das der Anschluss 10, 110 formschlüssig einsteckbar ist. Es kann auch eine magnetische Kupplung vorgesehen sein. Das Kantenloch 206 kann Teil einer Anschlussbuchse 208 sein, die starr mit einem Ende der beweglichen Welle 204 im Schlauch 202 verbunden ist. Am anderen Ende der beweglichen Welle 204 kann ein Verlängerungsanschluss 210 starr mit der beweglichen Welle 204 verbunden sein. Der Verlängerungsanschluss 210 kann mit einer externen Antriebseinheit verbunden sein oder verbunden werden und kann hierzu als Sechskant ausgeführt sein.

Figur 17 zeigt mit zwei schematischen perspektivischen Ansichten am Beispiel der dritten beispielhaften chirurgischen Reinigungsvorrichtung 101, wie die Antriebswellenverlängerung 200 mit dem Anschluss 110 der Antriebswelle 103 verbunden sein beziehungsweise verbunden werden kann. Das Beispiel ist ohne weiteres auch auf andere chirurgische Reinigungsvorrichtungen mit einem entsprechenden Anschluss übertragbar, wie beispielsweise der ersten beispielhaften chirurgischen Reinigungsvorrichtung 1 und der zweiten beispielhaften chirurgischen Reinigungsvorrichtung 51.

Die chirurgischen Reinigungsvorrichtungen 1, 51, 101 können mit und ohne Absaugeinrichtung 90, 140 vollständig aus Kunststoff gefertigt sein. Hierdurch können die erfindungsgemäßen chirurgischen Reinigungsvorrichtungen 1, 51, 101 nach Gebrauch zusammen mit anderem Abfall aus dem OP-Bereich entsorgt und auf hygienische vollständig Weise verbrannt werden. Gleichzeitig können die erfindungsgemäßen chirurgischen Reinigungsvorrichtungen 1, 51, 101 vor dem Gebrauch auf einfache Weise mit Hilfe von Ethylenoxid oder mit Gamma-Strahlen desinfiziert werden.

### Bezugszeichenliste

- 1, 51, 101: Chirurgische Reinigungsvorrichtung
- 2, 52, 102: Gehäuse
- 3, 53, 103: Antriebswelle
- 4, 104: Hinteres Ende der Antriebswelle
- 5, 55, 105: Vorderes Ende der Antriebswelle
- 6, 56, 106: Elastisch verformbare Borsten
- 7: Pumpe
- 8: Flüssigkeitszuleitung
- 9, 59, 109: Flüssigkeitsableitung
- 10, 110: Anschluss
- 11: Gegenlager
- 12: Pumpengehäuse
- 14: Anschluss
- 16: Lager
- 18: Pumpendeckel
- 20, 120: Lager
- 22, 72, 122: Wellengehäuse
- 24: Spritzschutz
- 26, 126: Kappe
- 27: Impellerflügel
- 28, 78, 128: Flüssigkeitsaustrittsöffnung
- 30: Spülflüssigkeit
- 32, 132: Blende
- 34: Dichtung
- 36: Aufnahme
- 38: Innengewinde
- 74: Vorsprung
- 90, 140: Absaugeinrichtung
- 91, 141: Absaugleitung
- 92, 142: Aufsteckbuchse
- 93, 143: Öffnung
- 94: Spritzschutz
- 95, 145: Ableitungsanschluss
- 96, 146: Spülflüssigkeitsableitung
- 97, 147: Absaugöffnung
- 98, 148: Innengewinde
- 134: Einsatz
- 144: Trichter
- 200: Antriebswellenverlängerung
- 202: Schlauch
- 204: Bewegliche Welle
- 206: Kantenloch
- 208: Anschlussbuchse
- 210: Verlängerungsanschluss

## Patentansprüche

1. Chirurgische Reinigungsvorrichtung (1, 51, 101) aufweisend
ein Gehäuse (2, 52, 102),
eine Antriebswelle (3, 53, 103), wobei die Antriebswelle (3, 53, 103) drehbar in dem Gehäuse (2, 52, 102) gelagert ist, wobei die Antriebswelle (3, 53, 103) ein hinteres Ende (4, 104) zur Verbindung mit einer Antriebseinheit aufweist und wobei die Antriebswelle (3, 53, 103) ein vorderes Ende (5, 55, 105) aufweist, das dem hinteren Ende (4, 104) gegenüberliegend angeordnet ist,
eine Vielzahl elastisch verformbarer Borsten (6, 56, 106), wobei die elastisch verformbaren Borsten (6, 56, 106) an dem vorderen Ende (5, 55, 105) der Antriebswelle (3, 53, 103) angeordnet sind und mit der Antriebswelle (3, 53, 103) verbunden sind, wobei die elastisch verformbaren Borsten (6, 56, 106) zumindest teilweise aus dem Gehäuse (2, 52, 102) hervorstehen und wobei die elastisch verformbaren Borsten (6, 56, 106) sich größtenteils oder alle von der Drehachse der Antriebswelle (3, 53, 103) weg erstrecken,
eine Pumpe (7) zum Pumpen einer Flüssigkeit, wobei die Pumpe (7) mit der Antriebswelle (3, 53, 103) verbunden ist und von der Antriebswelle (3, 53, 103) anzutreiben ist,
eine Flüssigkeitszuleitung (8), wobei die Flüssigkeitszuleitung (8) derart mit der Pumpe (7) verbunden ist, dass eine Flüssigkeit durch die Flüssigkeitszuleitung (8) von der Pumpe (7) in die Pumpe (7) ansaugbar ist,
eine Flüssigkeitsableitung (9, 59, 109), wobei die Flüssigkeitsableitung (9, 59, 109) derart mit der Pumpe (7) verbunden ist, dass eine Flüssigkeit von der Pumpe (7) aus der Pumpe (7) heraus in die Flüssigkeitszuleitung (8) drückbar ist,
wobei die Flüssigkeitsableitung (9, 59, 109) im Bereich der elastisch verformbaren Borsten (6, 56, 106) zumindest eine Flüssigkeitsaustrittsöffnung (28, 78, 128) aufweist, wobei die Flüssigkeitsableitung (9, 59, 109) durch die zumindest eine Flüssigkeitsaustrittsöffnung (28, 78, 128) in Richtung der elastisch verformbaren Borsten (6, 56, 106) mündet.

2. Chirurgische Reinigungsvorrichtung (1, 51, 101) nach Anspruch 1, **dadurch gekennzeichnet, dass**
das Gehäuse (2, 52, 102) ein Wellengehäuse (22, 72, 122) aufweist und ein Griffstück zum Halten der chirurgischen Reinigungsvorrichtung (1, 51, 101) mit einer Hand aufweist, wobei das Wellengehäuse (22, 72, 122) die Antriebswelle (3, 53, 103) zumindest in einem Bereich zwischen dem vorderen Ende (5, 55, 105) der Antriebswelle (3, 53, 103) und dem Griffstück umschließt und wobei das Wellengehäuse (22, 72, 122) mit dem Griffstück verbunden ist und das Griffstück eine Öffnung für das hinteren Ende (4, 104) der Antriebswelle (3, 53, 103) aufweist.

3. Chirurgische Reinigungsvorrichtung (1, 51, 101) nach Anspruch 1 oder 2, **dadurch gekennzeichnet, dass**
die Antriebswelle (3, 53, 103) durch eine separate Antriebseinheit drehbar und anzutreiben ist, wobei die separate Antriebseinheit hierzu mit dem hinteren Ende (4, 104) der Antriebswelle (3, 53, 103) verbunden oder verbindbar ist, insbesondere lösbar verbunden oder verbindbar ist.

4. Chirurgische Reinigungsvorrichtung (1, 51, 101) nach einem der vorangehenden Ansprüche, **dadurch gekennzeichnet, dass**
die elastisch verformbaren Borsten (6, 56, 106) sich axial und radial von der Antriebswelle (3, 53, 103) weg erstrecken und/oder
die elastisch verformbaren Borsten (6, 56, 106) den Außendurchmesser des Gehäuses (2, 52, 102) im Bereich der elastisch verformbaren Borsten (6, 56, 106) um mindestens 1 mm, bevorzugt 2 mm bis 4 mm und besonders bevorzugt um 5 mm bis 10 mm überragen.

5. Chirurgische Reinigungsvorrichtung (1, 51, 101) nach einem der vorangehenden Ansprüche, **dadurch gekennzeichnet, dass**
die Pumpe (7) eine selbstansaugende Pumpe ist, die unmittelbar mit der Antriebswelle (3, 53, 103) verbunden ist, wobei die selbstansaugende Pumpe in dem Gehäuse (2, 52, 102) angeordnet ist, und/oder
die Pumpe (7) derart mit der Antriebswelle (3, 53, 103) verbunden ist, dass zumindest bei einem Stillstand der Antriebswelle (3, 53, 103) die Flüssigkeitszuleitung (8) in der Pumpe (7) von der Flüssigkeitsableitung (9, 59, 109) flüssigkeitsundurchlässig getrennt ist oder abgeriegelt ist, wobei bevorzugt bei Drehbewegung der Antriebswelle (3, 53, 103) die Flüssigkeitszuleitung (8) flüssigkeitsleitend mit der Flüssigkeitsableitung (9, 59, 109) verbunden ist, und/oder
die Pumpe (7) eine Impellerpumpe ist, die auf der Antriebswelle (3, 53, 103) angeordnet ist, wobei die Impellerpumpe einen Rotor mit Impellerflügeln (27) aufweist, wobei der Rotor mit den Impellerflügeln (27) kraft- und/oder formschlüssig mit der Antriebswelle (3, 53, 103) verbunden ist.

6. Chirurgische Reinigungsvorrichtung (1, 51, 101) nach einem der vorangehenden Ansprüche, **dadurch gekennzeichnet, dass**
die Flüssigkeitsableitung (9, 59, 109) zumindest bereichsweise durch einen Teil des Gehäuses (2, 52, 102) gebildet ist, der einen Zwischenraum zwischen der Antriebswelle (3, 53, 103) und dem Gehäuse (2, 52, 102) begrenzt, wobei die zumindest eine Flüssigkeitsaustrittsöffnung (28, 78, 128) durch zumindest eine Bohrung in dem Gehäuse (2, 52, 102) oder in einer frontalen Dichtung des Gehäuses (2, 52, 102) im Bereich der elastisch verformbaren Borsten (6, 56, 106) realisiert ist.

7. Chirurgische Reinigungsvorrichtung (1, 51, 101) nach einem der vorangehenden Ansprüche, **dadurch gekennzeichnet, dass**
zumindest eine Blende (32, 132) auf der Antriebswelle (3, 53, 103) derart angebracht ist, so dass die zumindest eine Blende (32, 132) die zumindest eine Flüssigkeitsaustrittsöffnung (28, 78, 128) oder wenigstens eine von mehreren Flüssigkeitsaustrittöffnungen (28, 78, 128) bei einer vollständigen Drehung der Antriebswelle (3, 53, 103) um ihre Drehachse wenigstens einmal abdeckt und wenigstens einmal freigibt.

8. Chirurgische Reinigungsvorrichtung (1, 51, 101) nach einem der vorangehenden Ansprüche, **dadurch gekennzeichnet, dass**
die chirurgische Reinigungsvorrichtung (1, 51, 101) eine Absaugeinrichtung (90, 140) mit zumindest einer Absaugöffnung (97, 147) im Bereich der elastisch verformbaren Borsten (6, 56, 106) aufweist, wobei die Absaugeinrichtung (90, 140) eine Absaugleitung (91, 141) aufweist, die an eine Unterdruckquelle anschließbar ist oder angeschlossen ist, wobei
bevorzugt die Absaugleitung (91, 141) durch ein Rohr realisiert ist, wobei das Rohr das Gehäuse (2, 52, 102) zumindest abschnittsweise umschließt, und/oder
bevorzugt die Absaugeinrichtung (90, 140) an einen Abscheider zum Abscheiden von festen Bestandteilen aus der gebrauchten Spülflüssigkeit (30) angeschlossen oder anschließbar ist und/oder die Unterdruckquelle eine Vakuumpumpe aufweist, die ein Teil der chirurgischen Reinigungsvorrichtung (1, 51, 101) ist und die von der Antriebswelle (3, 53, 103) antreibbar ist.

9. Chirurgische Reinigungsvorrichtung (1, 51, 101) nach einem der vorangehenden Ansprüche, **dadurch gekennzeichnet, dass**
die chirurgische Reinigungsvorrichtung (1, 51, 101) einen Spritzschutz (24, 94) aufweist, wobei der Spritzschutz (24, 94) die elastisch verformbaren Borsten (6, 56, 106) teilweise abdeckt, wobei bevorzugt der Spritzschutz (24, 94) an dem Gehäuse (2, 52, 102) befestigt ist und entweder gegen das Gehäuse (2, 52, 102) drehbar gelagert ist oder starr mit dem Gehäuse (2, 52, 102) verbunden ist und/oder
die zumindest eine Flüssigkeitsaustrittsöffnung (28, 78, 128) derart geformt und derart relativ zu den elastisch verformbaren Borsten (6, 56, 106) angeordnet ist, dass ein durch die zumindest eine Flüssigkeitsaustrittsöffnung (28, 78, 128) ausgestoßener Strahl der Spülflüssigkeit (30) in den Wirkbereich der drehenden elastisch verformbaren Borsten (6, 56, 106) trifft.

10. Chirurgische Reinigungsvorrichtung (1, 51, 101) nach einem der vorangehenden Ansprüche, **dadurch gekennzeichnet, dass**
die chirurgische Reinigungsvorrichtung (1, 51, 101) aus Kunststoff gefertigt ist, wobei bevorzugt die gesamte chirurgische Reinigungsvorrichtung (1, 51, 101) durch Verbrennung rückstandsfrei entsorgbar ist.

11. Chirurgische Reinigungsvorrichtung (1, 51, 101) nach einem der vorangehenden Ansprüche, **dadurch gekennzeichnet, dass**
die chirurgische Reinigungsvorrichtung (1, 51, 101) eine flexible Antriebswellenverlängerung (200) aufweist, wobei die flexible Antriebswellenverlängerung (200) an das hintere Ende (4, 104) der Antriebswelle (3, 53, 103) angeschlossen oder anschließbar ist und wobei die flexible Antriebswellenverlängerung (200) an eine externe Antriebseinheit anschließbar ist, so dass die Antriebswelle (3, 53, 103) mit der externen Antriebseinheit über die flexible Antriebswellenverlängerung (200) gegen das Gehäuse (2, 52, 102) drehbar ist.

12. Nicht-chirurgisches und nicht-therapeutisches Verfahren zum Betreiben einer chirurgischen Reinigungsvorrichtung (1, 51, 101) wobei das Verfahren die folgenden Schritte aufweist:
A) Drehen einer Antriebswelle (3, 53, 103) der chirurgischen Reinigungsvorrichtung (1, 51, 101);
B) Drehen einer Vielzahl von elastisch verformbaren Borsten (6, 56, 106), die an einem vorderen Ende (5, 55, 105) der Antriebswelle (3, 53, 103) angeordnet sind, durch das Drehen der Antriebswelle (3, 53, 103);
C) Antreiben einer Pumpe (7) mit der sich drehenden Antriebswelle (3, 53, 103); und
D) Pumpen einer Spülflüssigkeit (30) mit der Pumpe (7) durch zumindest eine Flüssigkeitsaustrittsöffnung (28, 78, 128), wobei die Spülflüssigkeit (30) gepulst oder ungepulst in den Bereich der sich drehenden elastisch verformbaren Borsten (6, 56, 106) gesprüht wird, insbesondere in den Wirkbereich der sich drehenden elastisch verformbaren Borsten (6, 56, 106) gesprüht wird.

13. Verfahren nach Anspruch 12, **dadurch gekennzeichnet, dass**
das Verfahren mit einer chirurgischen Reinigungsvorrichtung (1, 51, 101) nach einem der Ansprüche 1 bis 11 durchgeführt wird.

14. Verfahren nach einem der Ansprüche 12 oder 13, **dadurch gekennzeichnet, dass**
vor Schritt A) die chirurgische Reinigungsvorrichtung (1, 51, 101) an eine Antriebseinheit angeschlossen wird, wobei die Drehung der Antriebswelle (3, 53, 103) in den Schritten A) bis D) mit der Antriebseinheit angetrieben wird, wobei bevorzugt die Antriebseinheit an ein dem vorderen Ende (5, 55, 105) gegenüberliegendes hinteres Ende (4, 104) der Antriebswelle (3, 53, 103) angeschlossen wird.

15. Verfahren nach einem der Ansprüche 12 bis 14, **dadurch gekennzeichnet, dass**
die gebrauchte Spülflüssigkeit (30) während der Schritte A) bis D) zumindest teilweise mit einer Absaugeinrichtung (90, 140) abgesaugt wird, wobei bevorzugt nach der Absaugung mit einem Abscheider feste Partikel der gebrauchten Spülflüssigkeit (30) von den flüssigen Bestandteilen getrennt werden.

## Claims

1. A surgical cleaning apparatus (1, 51, 101) comprising
a housing (2, 52, 102),
a drive shaft (3, 53, 103), wherein the drive shaft (3, 53, 103) is rotatably mounted in the housing (2, 52, 102), wherein the drive shaft (3, 53, 103) comprises a rear end (4, 104) for connecting to a drive unit, and wherein the drive shaft (3, 53, 103) comprises a front end (5, 55, 105) which is arranged opposite the rear end (4, 104),
a plurality of elastically deformable bristles (6, 56, 106), wherein the elastically deformable bristles (6, 56, 106) are arranged at the front end (5, 55, 105) of the drive shaft (3, 53, 103) and are connected to the drive shaft (3, 53, 103), wherein the elastically deformable bristles (6, 56, 106) protrude at least partially from the housing (2, 52, 102), and wherein most or all of the elastically deformable bristles (6, 56, 106) extend away from the axis of rotation of the drive shaft (3, 53, 103),
a pump (7) for pumping a liquid, wherein the pump (7) is connected to the drive shaft (3, 53, 103) and is to be driven by the drive shaft (3, 53, 103),
a liquid supply line (8), wherein the liquid supply line (8) is connected to the pump (7) in such a way that a liquid can be suctioned through the liquid supply line (8) and into the pump (7) by the pump (7),
a liquid discharge line (9, 59, 109), wherein the liquid discharge line (9, 59, 109) is connected to the pump (7) in such a way that a liquid can be forced out of the pump (7) and into the liquid supply line (8) by the pump (7),
wherein the liquid discharge line (9, 59, 109) comprises at least one liquid outlet opening (28, 78, 128) in the region of the elastically deformable bristles (6, 56, 106), wherein the liquid discharge line (9, 59, 109) opens in the direction of the elastically deformable bristles (6, 56, 106) through the at least one liquid outlet opening (28, 78, 128).

2. The surgical cleaning apparatus (1, 51, 101) according to claim 1, **characterized in that** the housing (2, 52, 102) comprises a shaft housing (22, 72, 122) and a handle for holding the surgical cleaning apparatus (1, 51, 101) using one hand, wherein the shaft housing (22, 72, 122) encloses the drive shaft (3, 53, 103) at least in a region between the front end (5, 55, 105) of the drive shaft (3, 53, 103) and the handle, and wherein the shaft housing (22, 72, 122) is connected to the handle, and the handle comprises an opening for the rear end (4, 104) of the drive shaft (3, 53, 103).

3. The surgical cleaning apparatus (1, 51, 101) according to claim 1 or claim 2,
**characterized in that**
the drive shaft (3, 53, 103) can be rotated and is to be driven by a separate drive unit, wherein the separate drive unit is or can be connected to the rear end (4, 104) of the drive shaft (3, 53, 103) for this purpose, in particular is detachably connected or detachably connectable.

4. The surgical cleaning apparatus (1, 51, 101) according to any of the preceding claims,
**characterized in that**
the elastically deformable bristles (6, 56, 106) extend axially and radially away from the drive shaft (3, 53, 103) and/or
the elastically deformable bristles (6, 56, 106) project beyond the outer diameter of the housing (2, 52, 102) in the region of the elastically deformable bristles (6, 56, 106) by at least 1 mm, preferably 2 mm to 4 mm and particularly preferably by 5 mm to 10 mm.

5. The surgical cleaning apparatus (1, 51, 101) according to any of the preceding claims,
**characterized in that**
the pump (7) is a self-priming pump which is directly connected to the drive shaft (3, 53, 103), wherein the self-priming pump is arranged in the housing (2, 52, 102), and/or
the pump (7) is connected to the drive shaft (3, 53, 103) in such a way that, at least when the drive shaft (3, 53, 103) is stationary, the liquid supply line (8) in the pump (7) is disconnected or sealed off from the liquid discharge line (9, 59, 109) in a liquid-impermeable manner, wherein the liquid supply line (8) is preferably connected to the liquid discharge line (9, 59, 109) in a liquid-conducting manner during rotational movement of the drive shaft (3, 53, 103), and/or
the pump (7) is an impeller pump which is arranged on the drive shaft (3, 53, 103), wherein the impeller pump comprises a rotor which has impeller blades (27), wherein the rotor which has the impeller blades (27) is non-positively and/or positively connected to the drive shaft (3, 53, 103).

6. The surgical cleaning apparatus (1, 51, 101) according to any of the preceding claims,
**characterized in that**
the liquid discharge line (9, 59, 109) is formed at least in regions by a portion of the housing (2, 52, 102) that delimits a space between the drive shaft (3, 53, 103) and the housing (2, 52, 102), wherein the at least one liquid outlet opening (28, 78, 128) is provided by at least one bore in the housing (2, 52, 102) or in a frontal seal of the housing (2, 52, 102) in the region of the elastically deformable bristles (6, 56, 106).

7. The surgical cleaning apparatus (1, 51, 101) according to any of the preceding claims,
**characterized in that**
at least one cover (32, 132) is mounted on the drive shaft (3, 53, 103) in such a way that the at least one cover (32, 132) covers and uncovers the at least one liquid outlet opening (28, 78, 128) or at least one of a plurality of liquid outlet openings (28, 78, 128) at least once during a complete rotation of the drive shaft (3, 53, 103) about its axis of rotation.

8. The surgical cleaning apparatus (1, 51, 101) according to any of the preceding claims,
**characterized in that**
the surgical cleaning apparatus (1, 51, 101) comprises a suction device (90, 140) which has at least one suction opening (97, 147) in the region of the elastically deformable bristles (6, 56, 106), wherein the suction device (90, 140) comprises a suction line (91, 141) which can be or is connected to a negative-pressure source,
wherein the suction line (91, 141) is preferably provided by a pipe, wherein the pipe encloses the housing (2, 52, 102) at least in portions, and/or
the suction device (90, 140) preferably is connected or connectable to a separator for separating solid components from the used rinsing liquid (30), and/or the negative-pressure source comprises a vacuum pump which is part of the surgical cleaning apparatus (1, 51, 101) and which is drivable by the drive shaft (3, 53, 103).

9. The surgical cleaning apparatus (1, 51, 101) according to any of the preceding claims,
**characterized in that**
the surgical cleaning apparatus (1, 51, 101) comprises a splash guard (24, 94), wherein the splash guard (24, 94) partially covers the elastically deformable bristles (6, 56, 106), wherein the splash guard (24, 94) is preferably fixed to the housing (2, 52, 102) and is either rotatably mounted with respect to the housing (2, 52, 102) or rigidly connected to the housing (2, 52, 102), and/or
the at least one liquid outlet opening (28, 78, 128) is shaped in such a way and arranged relative to the elastically deformable bristles (6, 56, 106) in such a way that a jet of rinsing liquid (30) expelled through the at least one liquid outlet opening (28, 78, 128) strikes the effective region of the rotating elastically deformable bristles (6, 56, 106).

10. The surgical cleaning apparatus (1, 51, 101) according to any of the preceding claims,
**characterized in that**
the surgical cleaning apparatus (1, 51, 101) is made of plastics material, wherein the entire surgical cleaning apparatus (1, 51, 101) can preferably be disposed of without residue by incineration.

11. The surgical cleaning apparatus (1, 51, 101) according to any of the preceding claims,
**characterized in that**
the surgical cleaning apparatus (1, 51, 101) comprises a flexible drive shaft extension (200), wherein the flexible drive shaft extension (200) is connected or connectable to the rear end (4, 104) of the drive shaft (3, 53, 103), and wherein the flexible drive shaft extension (200) can be connected to an external drive unit, so that the drive shaft (3, 53, 103) is rotatable with respect to the housing (2, 52, 102) via the flexible drive shaft extension (200) by means of the external drive unit.

12. A non-surgical and non-therapeutic method for operating a surgical cleaning apparatus (1, 51, 101),
the method comprising the following steps:
A) rotating a drive shaft (3, 53, 103) of the surgical cleaning apparatus (1, 51, 101);
B) rotating a plurality of elastically deformable bristles (6, 56, 106), which are arranged at a front end (5, 55, 105) of the drive shaft (3, 53, 103), by rotating the drive shaft (3, 53, 103);
C) driving a pump (7) using the rotating drive shaft (3, 53, 103); and
D) pumping a rinsing liquid (30), using the pump (7), through at least one liquid outlet opening (28, 78, 128), wherein the rinsing liquid (30) is sprayed in a pulsed or non-pulsed manner into the region of the rotating elastically deformable bristles (6, 56, 106), in particular is sprayed into the effective region of the rotating elastically deformable bristles (6, 56, 106).

13. The method according to claim 12, **characterized in that**
the method is carried out using a surgical cleaning apparatus (1, 51, 101) according to any of claims 1 to 11.

14. The method according to either claim 12 or claim 13, **characterized in that** before step A), the surgical cleaning apparatus (1, 51, 101) is connected to a drive unit, wherein the rotation of the drive shaft (3, 53, 103) in the steps A) to D) is driven by the drive unit, wherein the drive unit is preferably connected to a rear end (4, 104) of the drive shaft (3, 53, 103) opposite the front end (5, 55, 105).

15. The method according to any of claims 12 to 14, **characterized in that** the used rinsing liquid (30) is at least partially suctioned away using a suction device (90, 140) during the steps A) to D), wherein solid particles of the used rinsing liquid (30) are preferably separated from the liquid components using a separator after the suction.

## Revendications

1. Dispositif de nettoyage chirurgical (1, 51, 101) présentant
un boîtier (2, 52, 102),
un arbre d'entraînement (3, 53, 103), dans lequel l'arbre d'entraînement (3, 53, 103) est monté rotatif dans le boîtier (2, 52, 102), dans lequel l'arbre d'entraînement (3, 53, 103) présente une extrémité arrière (4, 104) pour la liaison à une unité d'entraînement et dans lequel l'arbre d'entraînement (3, 53, 103) présente une extrémité avant (5, 55, 105) disposée à l'opposé de l'extrémité arrière (4, 104),
une pluralité de poils élastiquement déformables (6, 56, 106), dans lequel les poils élastiquement déformables (6, 56, 106) sont disposés au niveau de l'extrémité avant (5, 55, 105) de l'arbre d'entraînement (3, 53, 103) et sont reliés à l'arbre d'entraînement (3, 53, 103), dans lequel les poils élastiquement déformables (6, 56, 106) font au moins partiellement saillie hors du boîtier (2, 52, 102) et dans lequel les poils élastiquement déformables (6, 56, 106) s'étendent, en grande partie ou en totalité, à distance de l'axe de rotation de l'arbre d'entraînement (3, 53, 103),
une pompe (7) permettant de pomper un liquide, dans lequel la pompe (7) est reliée à l'arbre d'entraînement (3, 53, 103) et doit être entraînée par l'arbre d'entraînement (3, 53, 103),
une conduite d'alimentation en liquide (8), dans lequel la conduite d'alimentation en liquide (8) est raccordée à la pompe (7) de telle sorte qu'un liquide peut être aspiré par la pompe (7) dans la pompe (7) en passant par la conduite d'alimentation en liquide (8), une conduite de dérivation de liquide (9, 59, 109), dans lequel la conduite de dérivation de liquide (9, 59, 109) est raccordée à la pompe (7) de telle sorte qu'un liquide peut être poussé par la pompe (7) hors de la pompe (7) dans la conduite d'alimentation en liquide (8),
dans lequel la conduite de dérivation de liquide (9, 59, 109) présente au moins une ouverture de sortie de liquide (28, 78, 128) dans la zone des poils élastiquement déformables (6, 56, 106), dans lequel la conduite de dérivation de liquide (9, 59, 109) débouche en direction des poils élastiquement déformables (6, 56, 106) à travers l'au moins une ouverture de sortie de liquide (28, 78, 128).

2. Dispositif de nettoyage chirurgical (1, 51, 101) selon la revendication 1, **caractérisé en ce que**
le boîtier (2, 52, 102) présente un boîtier pour arbre (22, 72, 122) et présente une pièce de préhension permettant de tenir le dispositif de nettoyage chirurgical (1, 51, 101) à une main, dans lequel le boîtier pour arbre (22, 72, 122) encercle l'arbre d'entraînement (3, 53, 103) au moins dans une zone entre l'extrémité avant (5, 55, 105) de l'arbre d'entraînement (3, 53, 103) et la pièce de préhension et dans lequel le boîtier pour arbre (22, 72, 122) est relié à la pièce de préhension et la pièce de préhension présente une ouverture pour l'extrémité arrière (4, 104) de l'arbre d'entraînement (3, 53, 103).

3. Dispositif de nettoyage chirurgical (1, 51, 101) selon la revendication 1 ou 2, **caractérisé en ce que**
l'arbre d'entraînement (3, 53, 103) est rotatif et doit être entraîné par une unité d'entraînement séparée, dans lequel l'unité d'entraînement séparée est à cet effet reliée ou peut être reliée, en particulier est reliée ou peut être reliée de manière amovible, à l'extrémité arrière (4, 104) de l'arbre d'entraînement (3, 53, 103).

4. Dispositif de nettoyage chirurgical (1, 51, 101) selon l'une des revendications précédentes, **caractérisé en ce que**
les poils élastiquement déformables (6, 56, 106) s'étendent axialement et radialement à distance de l'arbre d'entraînement (3, 53, 103) et/ou
les poils élastiquement déformables (6, 56, 106) dépassent d'au moins 1 mm, de préférence de 2 mm à 4 mm et de manière particulièrement préférée de 5 mm à 10 mm, du diamètre extérieur du boîtier (2, 52, 102) dans la zone des poils élastiquement déformables (6, 56, 106).

5. Dispositif de nettoyage chirurgical (1, 51, 101) selon l'une des revendications précédentes, **caractérisé en ce que**
la pompe (7) est une pompe autoamorçante qui est directement reliée à l'arbre d'entraînement (3, 53, 103), dans lequel la pompe autoamorçante est disposée dans le boîtier (2, 52, 102), et/ou
la pompe (7) est reliée à l'arbre d'entraînement (3, 53, 103) de telle sorte que, au moins lors d'un arrêt de l'arbre d'entraînement (3, 53, 103), la conduite d'alimentation en liquide (8) dans la pompe (7) est séparée de manière imperméable au liquide de la conduite de dérivation de liquide (9, 59, 109) ou est fermée, dans lequel, de préférence, lors du mouvement de rotation de l'arbre d'entraînement (3, 53, 103), la conduite d'alimentation en liquide (8) est raccordée de manière conductrice de liquide à la conduite de dérivation de liquide (9, 59, 109), et/ou
la pompe (7) est une pompe à aubes qui est disposée sur l'arbre d'entraînement (3, 53, 103), dans lequel la pompe à aubes présente un rotor comportant des pales d'aube (27), dans lequel le rotor comportant les pales d'aube (27) est relié par adhérence et/ou par complémentarité de forme à l'arbre d'entraînement (3, 53, 103).

6. Dispositif de nettoyage chirurgical (1, 51, 101) selon l'une des revendications précédentes, **caractérisé en ce que**
la conduite de dérivation de liquide (9, 59, 109) est formée au moins dans certaines zones par une partie du boîtier (2, 52, 102) qui délimite un espace intermédiaire entre l'arbre d'entraînement (3, 53, 103) et le boîtier (2, 52, 102), dans lequel l'au moins une ouverture de sortie de liquide (28, 78, 128) est réalisée par au moins un alésage dans le boîtier (2, 52, 102) ou dans un joint d'étanchéité frontal du boîtier (2, 52, 102) dans la zone des poils élastiquement déformables (6, 56, 106).

7. Dispositif de nettoyage chirurgical (1, 51, 101) selon l'une des revendications précédentes, **caractérisé en ce que**
au moins un diaphragme (32, 132) est monté sur l'arbre d'entraînement (3, 53, 103) de telle sorte que l'au moins un diaphragme (32, 132) recouvre au moins une fois et libère au moins une fois l'au moins une ouverture de sortie de liquide (28, 78, 128) ou au moins une ouverture de sortie de liquide parmi plusieurs ouvertures de sortie de liquide (28, 78, 128) lors d'une rotation complète de l'arbre d'entraînement (3, 53, 103) autour de son axe de rotation.

8. Dispositif de nettoyage chirurgical (1, 51, 101) selon l'une des revendications précédentes, **caractérisé en ce que**
le dispositif de nettoyage chirurgical (1, 51, 101) présente un appareil d'aspiration (90, 140) comportant au moins une ouverture d'aspiration (97, 147) dans la zone des poils élastiquement déformables (6, 56, 106), dans lequel l'appareil d'aspiration (90, 140) présente une conduite d'aspiration (91, 141) qui peut être raccordée ou est raccordée à une source de dépression, dans lequel
de préférence, la conduite d'aspiration (91, 141) est réalisée par un tube, dans lequel le tube encercle le boîtier (2, 52, 102) au moins dans certaines sections, et/ou de préférence, l'appareil d'aspiration (90, 140) est raccordé ou peut être raccordé à un séparateur permettant la séparation de composants solides du liquide de lavage (30) usagé et/ou la source de dépression présente une pompe à vide qui fait partie du dispositif de nettoyage chirurgical (1, 51, 101) et peut être entraînée par l'arbre d'entraînement (3, 53, 103).

9. Dispositif de nettoyage chirurgical (1, 51, 101) selon l'une des revendications précédentes, **caractérisé en ce que**
le dispositif de nettoyage chirurgical (1, 51, 101) présente une protection contre les éclaboussures (24, 94), dans lequel la protection contre les éclaboussures (24, 94) recouvre partiellement les poils élastiquement déformables (6, 56, 106), dans lequel, de préférence, la protection contre les éclaboussures (24, 94) est fixée au boîtier (2, 52, 102) et est soit montée de manière à pouvoir tourner contre le boîtier (2, 52, 102), soit reliée de manière rigide au boîtier (2, 52, 102), et/ou
l'au moins une ouverture de sortie de liquide (28, 78, 128) est formée et disposée par rapport aux poils élastiquement déformables (6, 56, 106) de telle sorte qu'un jet de liquide de lavage (30) éjecté à travers l'au moins une ouverture de sortie de liquide (28, 78, 128) atteint la zone d'action des poils élastiquement déformables (6, 56, 106) rotatifs.

10. Dispositif de nettoyage chirurgical (1, 51, 101) selon l'une des revendications précédentes, **caractérisé en ce que**
le dispositif de nettoyage chirurgical (1, 51, 101) est fabriqué en matière plastique, dans lequel, de préférence, l'ensemble du dispositif de nettoyage chirurgical (1, 51, 101) peut être éliminé sans résidus par incinération.

11. Dispositif de nettoyage chirurgical (1, 51, 101) selon l'une des revendications précédentes, **caractérisé en ce que**
le dispositif de nettoyage chirurgical (1, 51, 101) présente une extension d'arbre d'entraînement (200) flexible, dans lequel l'extension d'arbre d'entraînement (200) flexible est reliée ou peut être reliée à l'extrémité arrière (4, 104) de l'arbre d'entraînement (3, 53, 103) et dans lequel l'extension d'arbre d'entraînement (200) flexible peut être reliée à une unité d'entraînement externe de sorte que l'arbre d'entraînement (3, 53, 103) peut tourner contre le boîtier (2, 52, 102) avec l'unité d'entraînement externe par l'intermédiaire de l'extension d'arbre d'entraînement (200) flexible.

12. Procédé non chirurgical et non thérapeutique permettant de faire fonctionner un dispositif de nettoyage chirurgical (1, 51, 101)
dans lequel le procédé présente les étapes suivantes :
A) rotation d'un arbre d'entraînement (3, 53, 103) du dispositif de nettoyage chirurgical (1, 51, 101) ;
B) rotation d'une pluralité de poils élastiquement déformables (6, 56, 106) disposés au niveau d'une extrémité avant (5, 55, 105) de l'arbre d'entraînement (3, 53, 103) en faisant tourner l'arbre d'entraînement (3, 53, 103) ;
C) entraînement d'une pompe (7) avec l'arbre d'entraînement (3, 53, 103) rotatif ; et
D) pompage d'un liquide de lavage (30) avec la pompe (7) à travers au moins une ouverture de sortie de liquide (28, 78, 128), dans lequel le liquide de lavage (30) est pulvérisé de manière pulsée ou non pulsée dans la zone des poils élastiquement déformables (6, 56, 106) rotatifs, en particulier est pulvérisé dans la zone d'action des poils élastiquement déformables (6, 56, 106) rotatifs.

13. Procédé selon la revendication 12, **caractérisé en ce que**
le procédé est exécuté avec un dispositif de nettoyage chirurgical (1, 51, 101) selon l'une des revendications 1 à 11.

14. Procédé selon l'une des revendications 12 ou 13, **caractérisé en ce que**
avant l'étape A), le dispositif de nettoyage chirurgical (1, 51, 101) est relié à une unité d'entraînement, dans lequel la rotation de l'arbre d'entraînement (3, 53, 103) dans les étapes A) à D) est entraînée par l'unité d'entraînement, dans lequel, de préférence, l'unité d'entraînement est reliée à une extrémité arrière (4, 104) de l'arbre d'entraînement (3, 53, 103) opposée à l'extrémité avant (5, 55, 105).

15. Procédé selon l'une des revendications 12 à 14, **caractérisé en ce que** le liquide de lavage (30) usagé est aspiré au moins partiellement pendant les étapes A) à D) avec un appareil d'aspiration (90, 140), dans lequel, de préférence, après l'aspiration, des particules solides du liquide de lavage (30) usagé sont séparées des composants liquides avec un séparateur.
